(19) Europäisches Patentamt
European Patent Office
Office européen des brevets

(11) Veröffentlichungsnummer: **0 201 829**
**A1**

(12) **EUROPÄISCHE PATENTANMELDUNG**

(21) Anmeldenummer: **86106115.8**

(22) Anmeldetag: **05.05.86**

(51) Int. Cl.4: **C 07 D 215/56**
C 07 D 401/04, C 07 D 401/02
C 07 D 401/14, C 07 C 79/46
C 07 C 79/36, A 23 K 1/00
A 61 K 31/47

(30) Priorität: **15.05.85 DE 3517535**

(43) Veröffentlichungstag der Anmeldung:
**20.11.86 Patentblatt 86/47**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Grohe, Klaus, Dr.**
**Am Wasserturm 10**
**D-5068 Odenthal(DE)**

(72) Erfinder: **Zeiler, Hans-Joachim, Dr.**
**Elsbeekerstrasse 46**
**D-5620 Velbert 15(DE)**

(72) Erfinder: **Metzger, Karl Georg, Dr.**
**Pahlkestrasse 75**
**D-5600 Wuppertal(DE)**

(54) **1-Aryl-4-chinolon-3-carbonsäuren.**

(57) Die Erfindung betrifft neue 1-Aryl-4-chinolon-3-carbonsäuren der Formel (I)

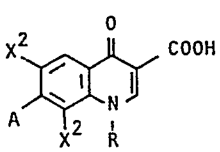

(I)

in welcher
$X^1$, $X^2$ und R die in der Beschreibung angegebene Bedeutung haben, Verfahren zu ihrer Herstellung sowie diese Verbindungen enthaltende antibakterielle Mittel und Futterzusatzstoffe.

BAYER AKTIENGESELLSCHAFT 5090 Leverkusen, Bayerwerk
Konzernverwaltung RP
Patentabteilung Ad/bo/c

1-Aryl-4-chinolon-3-carbonsäuren

Die Erfindung betrifft neue 1-Aryl-4-chinolon-3-carbonsäuren, Verfahren zu ihrer Herstellung sowie diese Verbindungen enthaltende antibakterielle Mittel und Futterzusatzstoffe.

Es wurde gefunden, daß die neuen 1-Aryl-4-chinolon-3-carbonsäuren der Formel (I),

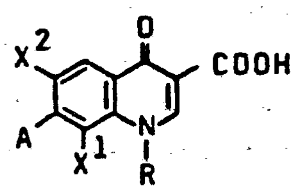

(I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen, Alkoxy.

Alkylmercapto oder Alkylsulfonyl mit jeweils bis zu 3 Kohlenstoffatomen, Nitro, Cyano, Carboxyl, Methylendioxy sowie einen Aminrest

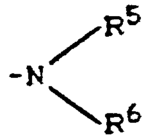 ,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 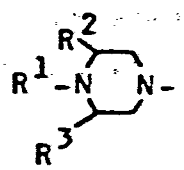 oder 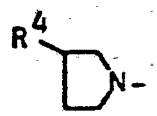 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 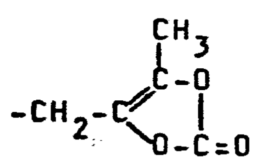 ,

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

und deren pharmazeutisch verwendbare Hydrate, Säureadditionssalze, Alkali-, Erdalkali-, Silber- und Guanidiniumsalze, sowie in Form ihrer Ester und in anderen üblichen Prodrug-Formen eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind diejenigen Verbindungen der Formel (I), in denen

$X^1$ für Chlor, Fluor oder Nitro,

$X^2$ für Chlor oder Fluor,

R für einen gegebenenfalls durch Halogen, Alkyl, Alkoxy, Alkylmercapto oder Alkylsulfonyl mit jeweils bis zu 2 Kohlenstoffatomen im Alkylteil, Nitro, Cyano substituierten Phenylrest oder einen Pyridin-, Thiophen-,Furan- oder Thiazolrest steht und

A für 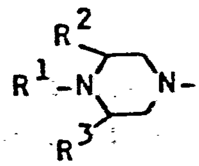 oder 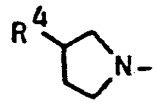 oder Halogen,

insbesondere Chlor oder Fluor steht, worin

$R^1$ für Wasserstoff, eine verzweigte oder unverzweigte Alkylgruppe mit 1 bis 3 Kohlenstoffatomen, die gegebenenfalls durch eine Hydroxygruppe substituiert sein kann, einen gegebenenfalls durch Chlor oder Fluor substituierten Phenacylrest, einen Oxoalkylrest mit 3 oder 4 Kohlenstoffatomen, 4-Aminobenzyl, Formyl oder Acetyl,

$R^2$ für Wasserstoff, Methyl oder gegebenenfalls durch Chlor oder Fluor substituiertes Phenyl,

$R^3$ für Wasserstoff oder Methyl und

$R^4$ für Wasserstoff, Hydroxy, Amino, Methylamino, Ethylamino, Aminomethyl, Methylaminomethyl, Ethylaminomethyl oder Diethylaminomethyl steht.

Besonders bevorzugt sind diejenigen Verbindungen der Formel (I), in denen

$X^1$ für Chlor oder Fluor,

$X^2$ für Fluor,

R für einen gegebenenfalls durch Chlor oder Fluor, Methyl, Methoxy, Methylmercapto, Nitro, Cyano substituierten Phenylrest steht, und

A für 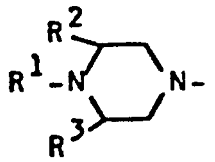 oder 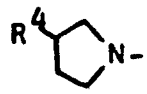 oder Halogen,

insbesondere Chlor oder Fluor steht, worin

$R^1$ für Wasserstoff, Methyl, Ethyl, 2-Hydroxyethyl, Phenacyl, 2-Oxopropyl, 3-Oxobutyl oder Formyl,

$R^2$ für Wasserstoff, Methyl oder Phenyl,

$R^3$ für Wasserstoff oder Methyl und

$R^4$ für Wasserstoff, Amino, Methylamino, Aminomethyl, Ethylaminomethyl oder Diethylaminomethyl steht.

Bevorzugt sind im übrigen die Verbindungen der Formel (I) in Form ihrer Methyl-, Ethyl-, Pivaloyloxymethyl-, Pivaloyloxyethyl- oder (5-Methyl-2-oxo-1,3-dioxol-4-yl-methyl)-ester.

Weiterhin wurde gefunden, daß man die Verbindungen der Formel (I) erhält, wenn man die 7-Halogen-4-chinolon-3-carbonsäuren der Formel (II),

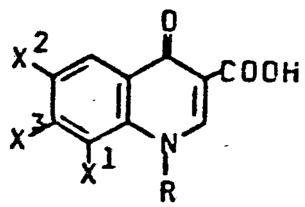

(II)

in welcher

R, $X^1$ und $X^2$ die oben angegebene Bedeutung haben und

$X^3$ für Halogen, bevorzugt Chlor oder Fluor steht,

mit Aminen der Formel (III),

A - H (III)

in welcher

A die oben angegebene Bedeutung hat,

gegebenenfalls in Gegenwart von Säurebindern umsetzt (Methode A).

Erfindungsgemäße Verbindungen der Formel (I) können auch erhalten werden, indem man 1,4-Dihydro-4-oxo-7-(1-piperazinyl)-chinolin-3-carbonsäuren der Formel (IV),

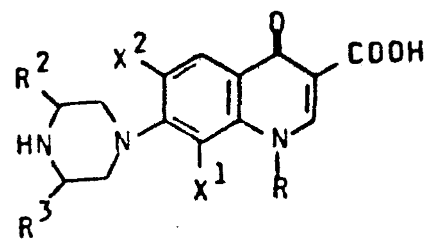

(IV)

in welcher

$X^1$, $X^2$, R, $R^2$ und $R^3$ die oben angegebene Bedeutung haben, mit Verbindungen der Formel (V),

$$R^1 - Z \qquad (V)$$

in welcher

$R^1$ die oben angegebene Bedeutung hat, jedoch nicht Wasserstoff sein kann, und

Z Halogen, insbesondere Chlor, Brom oder Iod, Acyloxy, Ethoxy oder Hydroxy

bedeutet,

gegebenenfalls in Gegenwart von Säurebindern umsetzt (Methode B).

Man erhält erfindungsgemäße Verbindungen der Formel (I) ($R^1$=$CH_3$-CO-$CH_2CH_2$-) auch, wenn man eine Verbindung der Formel (IV) mit Methylvinylketon der Formel (VI)

$$CH_3 - CO - CH = CH_2 \qquad (VI)$$

umsetzt (Methode C).

Verwendet man bei der Umsetzung nach Methode A 1-Methylpiperazin und 6,7,8-Trifluor-1,4-dihydro-4-oxo-1-(4-fluorphenyl)-chinolin-3-carbonsäure als Ausgangsstoffe, so kann der Reaktionsablauf durch das folgende Formelschema wiedergegeben werden:

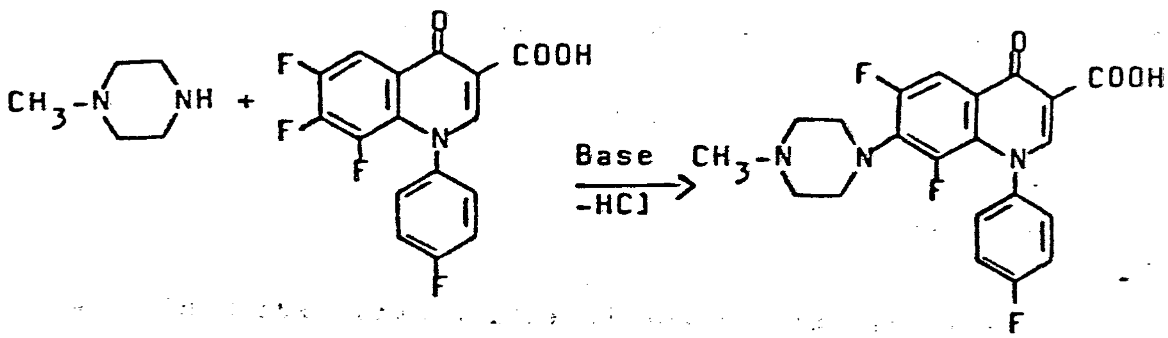


Verwendet man beispielsweise bei der Umsetzung nach Methode B Chloraceton und 6,8-Difluor-1,4-dihydro-4-oxo-1-(4-fluorphenyl)-7-(1-piperazinyl)-chinolin-3-carbonsäure als Ausgangsstoffe, so kann der Reaktionsablauf durch das folgende Formelschema wiedergegeben werden:

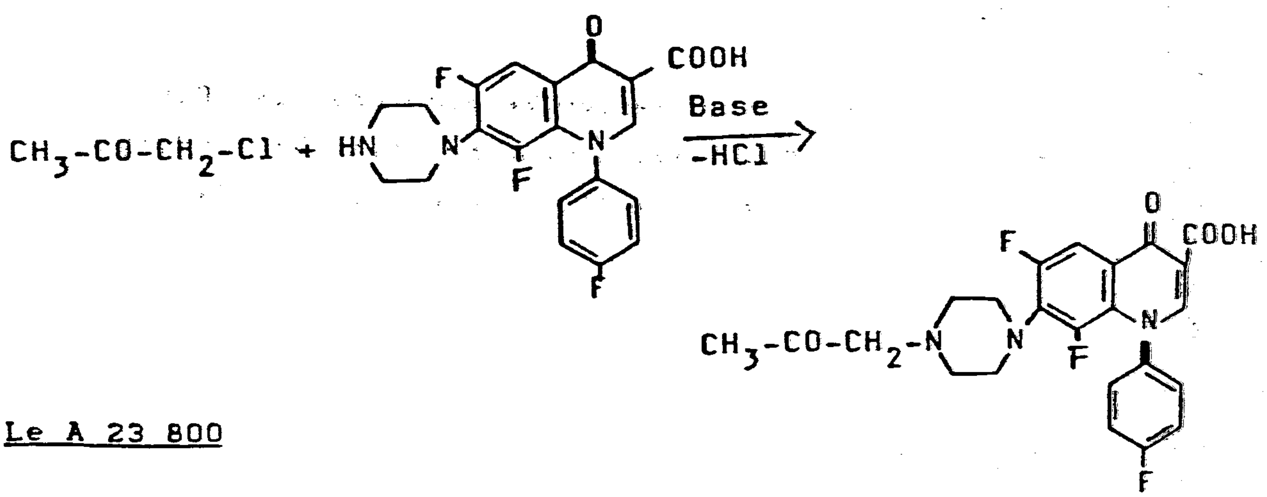

Verwendet man beispielsweise nach Methode C Methylvinylketon und 6,8-Difluor-1,4-dihydro-4-oxo-1-(4-fluorphenyl)-7-(1-piperazinyl)-chinolin-3-carbonsäure als Ausgangsverbindungen, so kann der Reaktionsablauf durch das folgende Formelschema wiedergegeben werden:

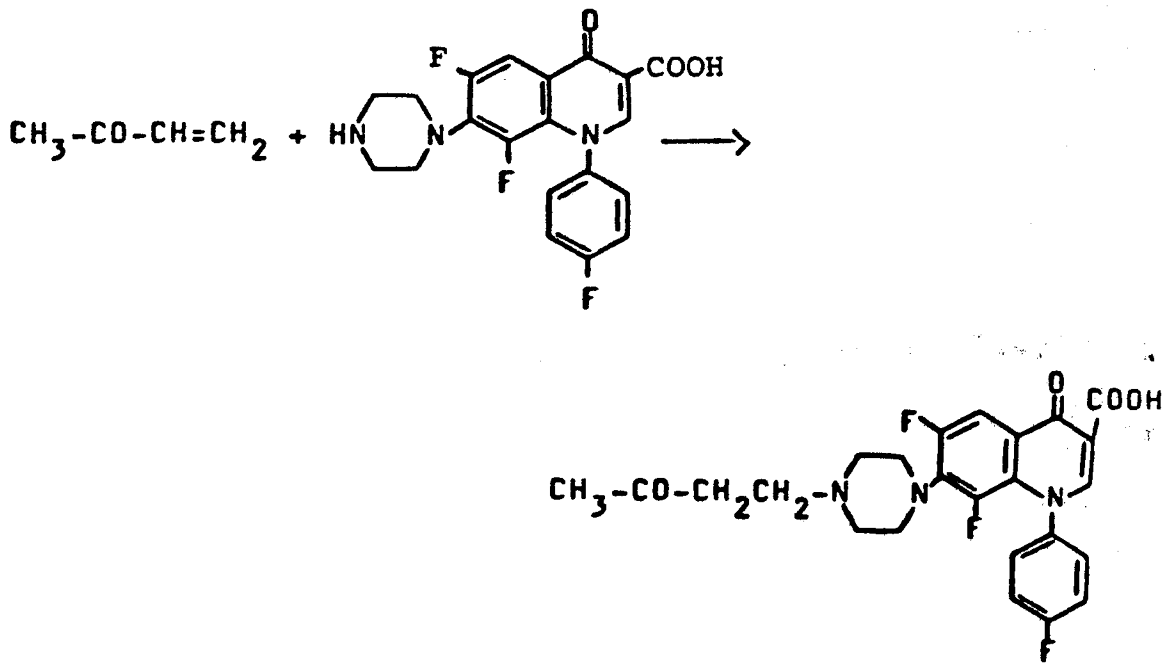

Die als Ausgangsstoffe nach Methode A verwendeten 7-Halogen-4-chinolon-3-carbonsäuren der Formel (II) können gemäß folgendem Reaktionsschema hergestellt werden:

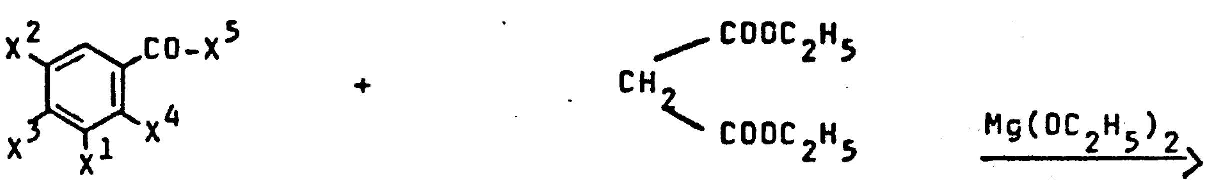


(1), $X^4$, $X^5$ = Cl, Br oder F (2)

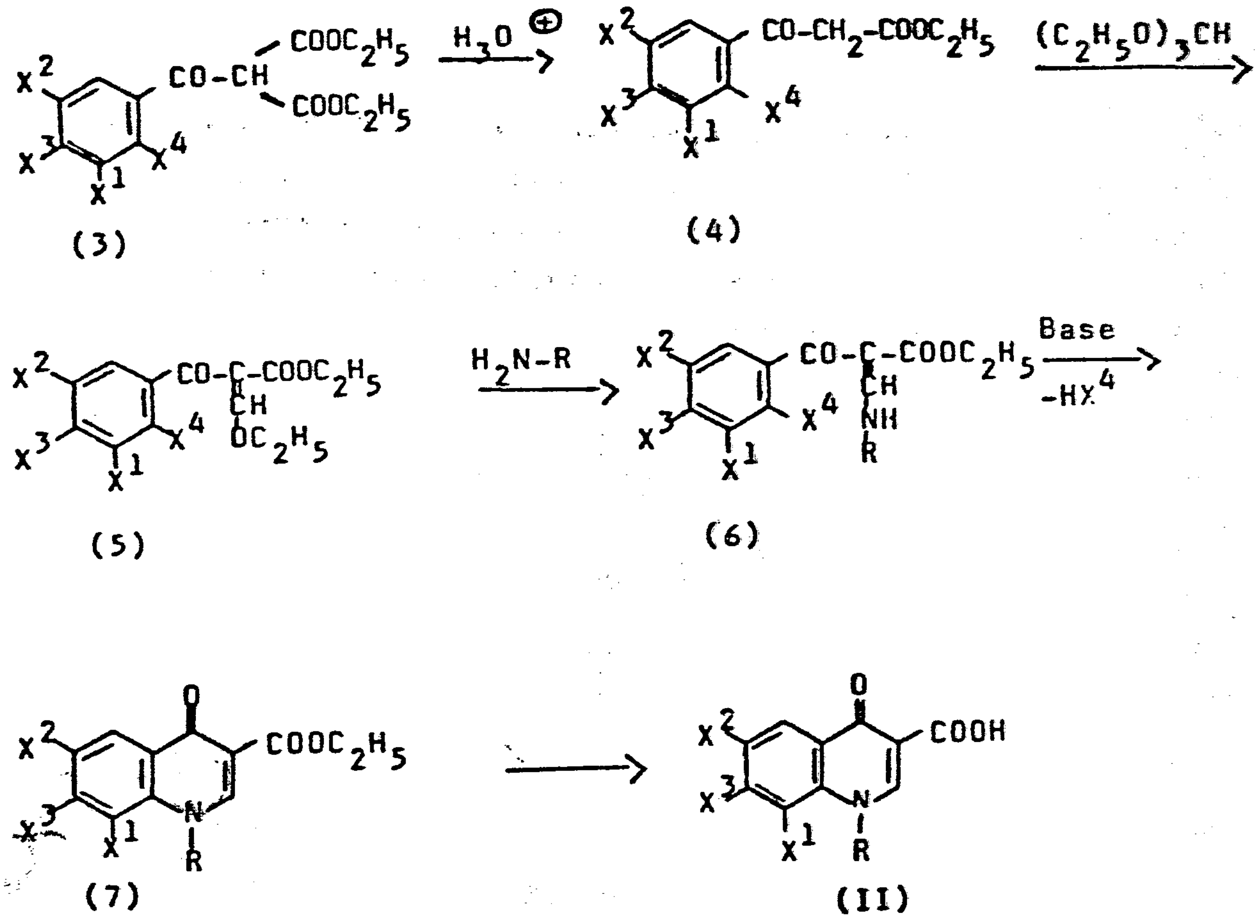


(3) (4)

(5) (6)

(7) (II)

Danach wird Malonsäurediethylester (2) in Gegenwart von Magnesiumethylat mit dem entsprechenden Benzoylhalogenid (1) zum Acylmalonester (3) acyliert (Organicum, 3. Auf. 1964. S. 438).

Durch partielle Verseifung und Decarboxylierung von (3) in wäßrigem Medium mit katalytischen Mengen Schwefelsäure

oder 4-Toluolsulfonsäure erhält man in guter Ausbeute den Acylessigsäureethylester (4), der mit Orthoameisensäure-triethylester/Acetanhydrid in den 2-Benzoyl-3-ethoxy-acrylsäureethylester (5) übergeht. Die Umsetzung von (5) mit dem Amin R-$NH_2$ in einem Lösungsmittel, wie z.B. Methylenchlorid, einem Alkohol, Chloroform, Cyclohexan oder Toluol führt in leicht exothermer Reaktion zu den gewünschten Zwischenprodukten (6).

Die Cyclisierungsreaktion (6) → (7) wird in einem Temperaturbereich von eta 60 bis 300°C, bevorzugt 80 bis 180°C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Als Säurebinder kommen für diese Reaktionsstufe Kalium-tert.-butanolat, Butyl-lithium, Lithium-phenyl, Phenylmagnesiumbromid, Natriummethylat, Natriumhydrid, Natrium- oder Kaliumcarbonat in Betracht. Besonders bevorzugt werden Kalium- oder Natriumfluorid, wenn Fluorwasserstoff abgespalten werden muß.

Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zu den 4-Chinolon-3-carbonsäuren (II).

Das als Ausgangsstoff für diesen Syntheseweg verwendete 2,3,4,5-Tetrafluorbenzoylchlorid ist bereits bekannt. 3,5-Dichlor-2,4-difluor-benzoylfluorid (Siedepunkt 97°/20 mbar; $n_D^{20}$ = 1,5148) und 5-Chlor-2,3,4-trifluor-benzoylfluorid (Siedepunkt 66-70°/20 mbar; $n_D^{20}$ = 1,4764) erhält man nebeneinander beim Erhitzen von Tetrachlor-benzoylchlorid mit Kaliumfluorid in Sulfolan auf erhöhte Temperaturen:

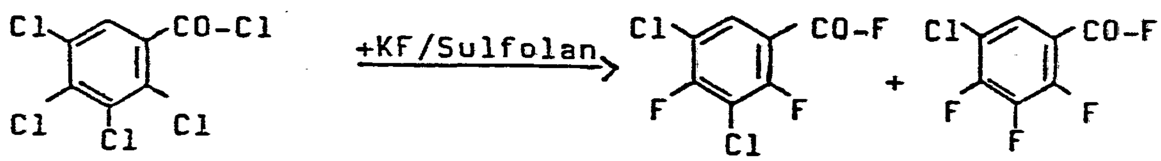


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94°/18 mbar; $n_D^{20}$ = 1,5164) umgesetzt wird:

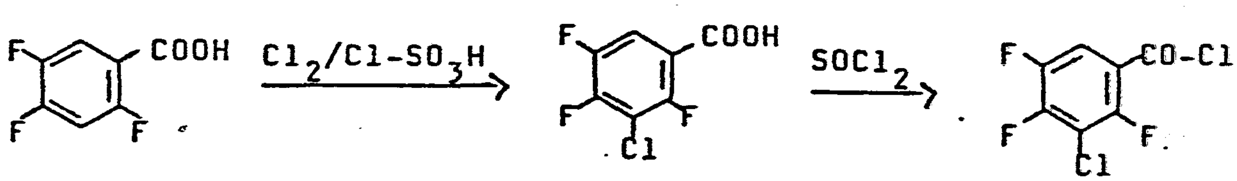


Das 2,4-Dichlor-5-fluor-3-nitro-benzoylchlorid wird durch Nitrierung der bekannten 2,4-Dichlor-5-fluor-benzoesäure zur 2,4-Dichlor-5-fluor-3-nitro-benzoesäure und deren Umsetzung mit Thionylchlorid erhalten.

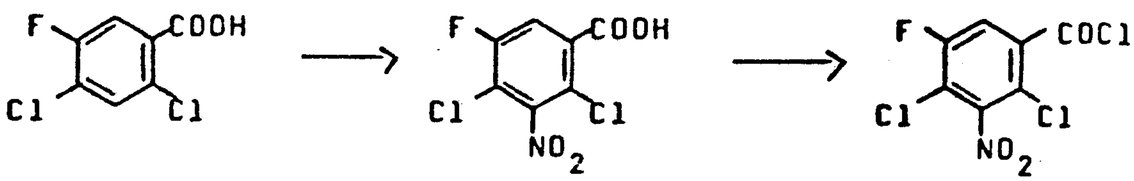

Die als Ausgangsstoffe verwendeten Amine der Formel (III) sind bekannt [US 4 166 180, J. Med. Chem. 26, 1116 (1983)]. Als Beispiele seien genannt: Piperazin, N-Methylpiperazin, N-Ethylpiperazin, N-(2-Hydroxyethyl)-piperazin, N-(2-Methoxyethyl)-piperazin, N-Propylpiperazin, N-Isopropylpiperazin, N-Butylpiperazin, N-(sek.-Butyl)-piperazin. N-Formylpiperazin, 2-Methylpiperazin, cis- und trans-2,6-Dimethylpiperazin, 2-Phenylpiperazin, 2-(4-Fluorphenyl)-piperazin, 2-(4-Chlorphenyl)-piperazin, 2-(4-Methylphenyl)-piperazin, 2-(4-Methoxyphenyl)-piperazin, 2-(4-Hydroxyphenyl)-piperazin, 2-(2-Thienyl)-piperazin, Pyrrolidin, 3-Amino-pyrrolidin, 3-Methylamino-pyrrolidin, 3-Aminomethyl-pyrrolidin, 3-Methylaminomethyl-pyrrolidin, 3-Dimethylaminomethyl-pyrrolidin, 3-Ethylaminomethyl-pyrrolidin, 3-Hydroxy-pyrrolidin.

Die als Ausgangsstoffe verwendeten Verbindungen der Formel (V) sind bekannt. Als Beispiele seien genannt: Methyliodid, Methylbromid, Ethyliodid, Ethylbromid, 2-Hydroxyethylchlorid, 3-Hydroxypropylchlorid, n-Propylbromid, Isopropyliodid, n-Butylbromid, sek.-Butyliodid, Isobutylbromid, Ameisensäure-essigsäure-anhydrid, Ameisensäureethylester, Ameisensäure, Essigsäureanhydrid, Acetylchlorid.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]-octan (DABCO), 1,8-Diazabicyclo[5,4,0]-undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol, des Amins (III) ein.

Freie Aminogruppen können während der Umsetzung durch eine geeignete Aminoschutzgruppe, zum Beispiel der t-Butoxycarbonyl-, Ethoxycarbonyl- oder Acetylgruppe geschützt, und nach Beendigung der Reaktion wieder entfernt werden. Eine aromatische Aminogruppe wird über die Reduktion einer Nitrogruppe eingeführt.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-tris-amid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo-[2,2,2]-octan (DABCO) oder 1,8-Diazabicyclo-[5,4,0]undec-7-en (DBU).

Die Reaktionstemperaturen können in einem größeren Bereich variiert werden. Im allgemeinen arbeitet man zwischen etwa 20 und etwa 180°C, vorzugsweise zwischen 40 und 110°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol, der Verbindung (V) ein.

Die Umsetzung von (IV) mit (VI) (Methode C) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem größeren Bereich variiert werden. Im allgemeinen arbeitet man zwischen etwa 20°C und etwa 150°C, vorzugsweise zwischen 50°C und 100°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens nach Methode C setzt man auf 1 Mol der Verbindung (IV) 1 bis 5 Mol, vorzugsweise 1 bis 2 Mol, der Verbindung (VI) ein.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel (Methanol, Ethanol, Aceton, Acetonitril). Man kann auch äquivalente Mengen Betain und

Säure in Wasser bis zur Lösung erhitzen und anschließend bis zur Trockne eindampfen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, Galacturonsäure, Gluconsäure, Glutaminsäure oder Asparaginsäure zu verstehen.

Die Alkali- oder Erdalkalisalze werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze der 1,4-Dihydro-4-oxo-chinolin-3-carbonsäuren erhalten.

Außer den in den Beispielen aufgeführten Verbindungen seien als neue Wirkstoffe in einzelnen genannt: 6,8-Difluor-1,4-dihydro-4-oxo-1-(4-fluorphenyl)-7-(3,5-dimethyl-1-piperazinyl)-chinolin-3-carbonsäure, 6,8-Difluor-1,4-dihydro-4-oxo-1-(3-fluorphenyl)-7-(3,5-dimethyl-1-piperazinyl)-chinolin-3-carbonsäure, 6,8-Difluor-1,4-dihydro-4-oxo-1-(2-fluorphenyl)-7-(3,5-dimethyl-1-piperazinyl)-chinolin-3-carbonsäure, 6,8-Difluor-1,4-dihydro-4-oxo-1-(2,4-difluorphenyl)-7-(1-piperazinyl)-chinolin-3-carbonsäure, 6,8-Difluor-1,4-dihydro-4-oxo-1-(2,4-difluorphenyl)-7-(4-methyl-1-piperazinyl)-chinolin-3-carbonsäure, 6,8-Difluor-1,4-dihydro-4-oxo-(2,4-difluorphenyl)-7-(3-methyl-1-piperazinyl)-chinolin-3-carbonsäure,

6,8-Difluor-1,4-dihydro-4-oxo-1-(2,4-difluorphenyl)-7-pyrrolidinyl-chinolin-3-carbonsäure, 6,8-Difluor-1,4-dihydro-4-oxo-1-(2,4-difluorphenyl)-7-(3-amino-1-pyrrolidinyl)-chinolin-3-carbonsäure, 6,8-Difluor-1,4-dihydro-4-oxo-1-(2,4-difluor-phenyl)-7-(3-methylamino-1-pyrrolidinyl)-chinolin-3-carbonsäure, 6,8-Difluor-1,4-dihydro-4-oxo-1-(2,4-difluorphenyl)-7-(3-ethylamino-1-pyrrolidinyl)-chinolin-3-carbonsäure, 6,8-Difluor-1,4-dihydro-4-oxo-1-(2,4-difluorphenyl)-7-(3-dimethylamino-1-pyrrolidinyl)-chinolin-3-carbonsäure, 6,8-Difluor-1,4-dihydro-4-oxo-1-(2,4-difluorphenyl)-7-(3-ethylamino-methyl-1-pyrrolidinyl)-chinolin-3-carbonsäure, 8-Chlor-6-fluor-1,4-dihydro-4-oxo-1-phenyl-7-(1-piperazinyl)-chinolin-3-carbonsäure, 8-Chlor-6-fluor-1,4-dihydro-4-oxo-1-phenyl-7-(4-methyl-1-piperazinyl)-chinolin-3-carbonsäure, 8-Chlor-6-fluor-1,4-dihydro-4-oxo-1-phenyl-7-(3-methyl-1-piperazinyl)-chinolin-3-carbonsäure, 8-Chlor-6-fluor-1,4-dihydro-4-oxo-1-phenyl-7-pyrrolidinyl-chinolin-3-carbonsäure, 8-Chlor-6-fluor-1,4-dihydro-4-oxo-1-phenyl-7-(3-methylamino-1-pyrrolidinyl)-chinolin-3-carbonsäure, 8-Chlor-6-fluor-1,4-dihydro-4-oxo-1-phenyl-7-(3-ethylaminomethyl-1-pyrrolidinyl)-chinolin-3-carbonsäure, 6-Fluor-1,4-dihydro-8-nitro-4-oxo-1-phenyl-7-(4-ethyl-1-piperazinyl)-chinolin-3-carbonsäure.

Weiterer Gegenstand der Erfindung sind Verbindungen der Formel (VII)

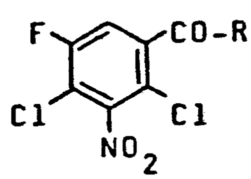

(VII)

in der

R Hydroxyl, Halogen, insbesondere Chlor, oder Alkoxycarbonylmethyl mit 1 - 4 Kohlenstoffatomen im Alkoxyteil bedeutet.

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykole (DAB) | 2,0 mg |
| Titan-(IV)-oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen

Enterobakteriaceen, vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staph. aureus, Staph. Epidermidis, (Staph. = Staphylococcus); Lactobacteriaceae, wie Streptokokken, z.B. Streptococcus pyogenes, α- bzw. β-hämolysierende Streptokokken, nicht-γ-hämolysierende Streptokokken, Enterokokken und Diplococcus pneumoniae (Pneumokokken), Enterobacteriaceae, wie Escherichiae-Bakterien der Escherichia-Gruppe, z.B. Escherichia coli, Enterobacter-Bakterien, z.B. E. aerogenes, E. cloacae (E. = Enterobacter), Klebsiella-Bakterien, z.B. K. pneumoniae (K. = Klebsiella), Serratia, z.B. Serratia marcescens, Proteae-Bakterien der Proteus-Gruppe: Proteus, z.B. Pr. vulgaris, Pr. morganii, Pr. rettgeri, Pr. mirabilis (Pr. = Proteus); Pseudomonadaceae, wie Pseudomonas-Bakterien, z.B. Ps. aeruginosa (Ps. = Pseudomonas); Bacteroidaceae, wie Bacteroides-Bakterien, z.B. Bacteroides fragilis; Mykoplasmen, z.B. Mykoplasma pneumonia, außerdem Mykobakterien, z.B. Mykobacterium tuberculosis, Mycobacterium leprae und atypische Mykobakterien.

Die obige Aufzählung von Erregern ist lediglich beispielhaft und keineswegs beschränkend aufzufassen. Als Krankheiten, die durch die erfindungsgemäßen Verbindungen verhindert, gebessert und/oder geheilt werden können, seien beispielsweise genannt:
Otitis; Pharyngitis; Pneumonie; Peritonitis; Pyelonephritis; Cystitis; Endocarditis; Systeminfektionen; Bronchitis; Arthritis; lokale Infektionen; septische Erkrankungen.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen

enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stcffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur Parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamktmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen angegeben.

**Tabelle**

| Stamm | Minimale Hemmkonzentration mcg/ml x) Beispiel Nr. 2 | Beispiel Nr. 3 | Beispiel Nr. 1 | Norfloxacin |
|---|---|---|---|---|
| E. coli 4418 | 0,125 | 0,06 | 0,25 | 0,125 |
| E. coli Neum. | ≤ 0,015 | ≤ 0,015 | 0,125 | 0,125 |
| E. coli 455/7 | 8 | 8 | 16 | 8 |
| Klebsiella pneum. 63 | 0,125 | 0,06 | 0,25 | 0,25 |
| Klebsiella pneum. 8085 | 0,06 | ≤ 0,015 | 0,125 | 0,25 |
| Klebsiella spec. 6179 | 0,25 | 0,06 | 0,5 | 2 |
| Proteus vulg. 1017 | 0,25 | 0,03 | 0,5 | 0,06 |
| Staphylococcus aur. 133 | 0,5 | 0,25 | 0,5 | 1 |
| Staphylococcus aur. 1756 | 0,5 | 0,25 | 0,5 | 1 |
| Streptococcus faecalis 27101 | 4,0 | 4,0 | 4,0 | 1 |
| Pseudomonas aerug. W. | 4,0 | 1 | 8 | 2 |

x) Agarverdünnungstest /Denley Multipoint - Inokulator
Isosensitestmedium

Die folgenden Beispiele erläutern die Erfindung:

Herstellung der Ausgangsverbindungen

Beispiel A

7-Chlor-6-fluor-1,4-dihydro-4-oxo-8-nitro-1-phenyl-chinolin-3-carbonsäure

A) 2,4-Dichlor-5-fluor-3-nitro-benzoesäure

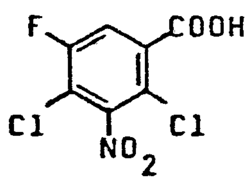

Unter Eiskühlung und Rühren werden 34 ml konz. Schwefelsäure tropfenweise mit 40 ml konz. Salpetersäure versetzt. In dieses Nitriergemisch trägt man portionsweise 20,9 g 2,4-Dichlor-5-fluorbenzoesäure ein, wobei die Temperatur auf 45 - 50°C steigt. Dann wird noch 3 Stunden auf 90 - 100°C erhitzt, das auf Raumtemperatur abgekühlte Gemisch auf 350 ml Eiswasser gegossen, der Niederschlag abgesaugt und mit Wasser gewaschen. Das feuchte Rohprodukt wird in 30 ml Methanol heiß gelöst und die Lösung mit 150 ml $H_2O$ versetzt.

Der Niederschlag wird kalt abgesaugt, mit $CH_3OH/H_2O$ gewaschen und i. Vak. bei 80°C getrocknet. Es werden 21,2 g rohe 2,4-Dichlor-5-fluor-3-nitro-benzoesäure

erhalten. Sie ist genügend rein für die weiteren Umsetzungen. Eine Probe aus Toluol/Petrolether umkristallisiert liefert Kristalle vom Schmelzpunkt 192°C.

b) 2,4-Dichlor-5-fluor-3-nitro-benzoylchlorid

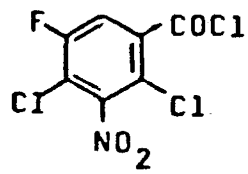

106,6 g 2,4-Dichlor-5-fluor-3-nitro-benzoesäure werden mit 250 ml Thionylchlorid 2 Stunden unter Rückfluß zum Sieden erhitzt. Das überschüssige Thionylchlorid wird dann bei Normaldruck abdestilliert und der Rückstand im Feinvak. fraktioniert. Bei 110 - 115°C/0,08-0,09 mbar gehen 104,7 g 2,4-Dichlor-5-fluor-3-nitro-benzoylchlorid über. Beim Stehen bilden sich Kristalle vom Schmelzpunkt 35 - 37°C.

c) (2,4-Dichlor-5-fluor-3-nitro-benzoyl)-essigsäureethylester

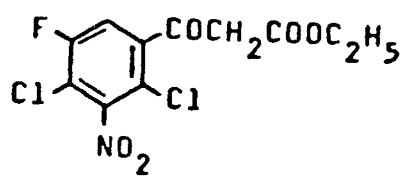

10,1 g Magnesiumspäne werden in 21 ml Ethanol mit 2,1 g Tetrachlormethan versetzt und nach Beginn der Wasserstoff-Entwicklung ein Gemisch aus 66,6 g Malonsäurediethylester, 40 ml Ethanol und 150 ml Toluol bei 50 - 60°C tropfenweise zugefügt. Man rührt 1 Stunde bei dieser Temperatur nach, kühlt auf -5 bis -10°C und tropft langsam eine Lösung von 109,2 g 2,4-Dichlor-5-fluor-3-nitro-benzoylchlorid in 50 ml Toluol zu. Danach wird 1 Stunde bei 0°C gerührt, über Nacht auf Raumtemperatur gebracht und noch 2 Stunden auf 40 - 50°C erwärmt. Das Reaktionsgemisch wird unter Eiskühlung mit einem Gemisch aus 160 ml Wasser und 10,4 ml konzentrierter Schwefelsäure versetzt und die organische Phase abgetrennt. Die wäßrige Phase wird mit Toluol extrahiert und der vereinigte organische Extrakt mit gesättigter Natriumchlorid-Lösung gewaschen, mit Natriumsulfat getrocknet und das Lösungsmittel abgezogen. Man erhält 144,5 g (2,4-Dichlor-5-fluor-3-nitro-benzoyl)-malonsäurediethylester als Rohprodukt. Dieses wird nach Zugabe von 200 ml Wasser und 0,6 g 4-Toluolsulfonsäure 3 Stunden unter Rückfluß erhitzt, die Mischung mit Methylenchlorid extrahiert, der Extrakt mit Natriumsulfat getrocknet und das Lösungsmittel im Vakuum abdestilliert. Es werden 118 g substituierter Benzoylessigester als Rohprodukt erhalten. Er besitzt eine für die weiteren Umsetzungen genügende Reinheit.

d) 7-Chlor-6-fluor-1,4-dihydro-4-oxo-8-nitro-1-phenyl-chinolin-3-carbonsäure

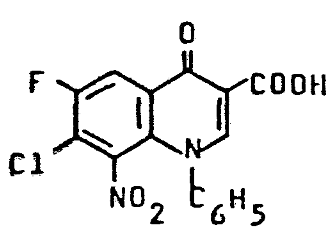

244,8 g (2,4-Dichlor-5-fluor-3-nitro-benzoyl)-essigsäureethylester werden mit 166 g Orthoameisensäuretriethylester und 185 g Essigsäureanhydrid 3 Stunden auf 150 -1 60°C erhitzt. Man engt im Vak. ein und erhält 270 g 2-(2,4-Dichlor-5-fluor-3-nitro-benzoyl)-3-ethoxy-acrylsäure-ethylester als öligen Rückstand.

38 g dieser Zwischenstufe werden in 100 ml Ethanol unter Eiskühlung tropfenweise mit 9,5 g Anilin versetzt und 1 Stunde bei 20°C gerührt. Das ausgefallene Produkt wird nach Zugabe von 100 ml Wasser abgesaugt, mit Ethanol/$H_2O$ (1:1) gewaschen und getrocknet. Man erhält 37,9 g 2-(2,4- Dichlor-5-fluor-3-nitro-benzoyl)-3-anilino-acrylsäure ethylester (6) ($R = C_6H_5$, $X^1 = NO_2$, $X^2 = F$, $X^3 = X^4 = Cl$) vom Schmelzpunkt 133 - 135°C.

37 g der vorgenannten Verbindung werden in 100 ml wasserfreiem Dioxan mit 13,3 g DBU versetzt und 4 Std. auf 100°C erhitzt. Das Lösungsmittel wird im Vakuum abdestilliert, der Rückstand in Methylenchlorid/Wasser aufgenommen, die Methylenchlorid-Phase abgetrennt, mit Natriumsulfat getrocknet und das Methylenchlorid abdestilliert. Es werden 32 g 7-Chlor-6-fluor-1,4-dihydro-4-oxo-8-nitro-1-phenyl-chinolin-3-carbonsäureethylester als Rohprodukt erhalten. Nach Umkristallisation aus Ethanol schmelzen die hellbraunen Kristalle bei 186 - 188°C.

10,5 g dieses Esters werden in einem Gemisch aus 100 ml Essigsäure, 70 ml Wasser und 10 ml konzentrierter

Schwefelsäure 2 Std. auf 150°C erhitzt. Die Suspension wird in 300 ml Eiswasser gegossen, der Niederschlag abgesaugt, mit Wasser und Methanol gewaschen und im Vakuum getrocknet.
Ausbeute: 7,5 g 7-Chlor-6-fluor-1,4-dihydro-4-oxo-8-nitro- 1-phenyl-chinolin-3-carbonsäure vom Schmelzpunkt 249 - 250°C.

Beispiel B

6,7,8-Trifluor-1,4-dihydro-4-oxo-1-(4-fluorphenyl)-chinolin-3-carbonsäure

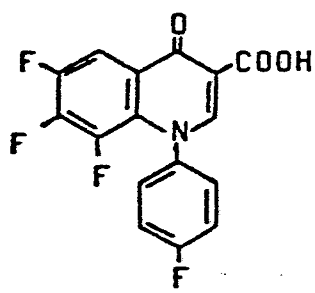

a) Eine Lösung von 16 g 3-Ethoxy-2-(2,3,4,5-tetra-fluorbenzoyl)-acrylsäureethylester (5) ($X^1$-$X^4$=F) (Le A 22 302) in 50 ml Ethanol wird unter Eiskühlung und Rühren tropfenweise mit 5,7 g 4-Fluoranilin versetzt. Man rührt 1 Stunde bei Raumtemperatur, gibt 50 ml Eiswasser zu, saugt den Niederschlag kalt ab, wäscht mit Wasser nach und trocknet im Vak. bei 50°C über Calciumchlorid. Es werden 16,5 g (6) (R = 4-Fluorphenyl, $X^1$-$X^4$ = F) vom Schmelzpunkt 93 - 95°C erhalten.

b) Eine Lösung von 16 g 2-(2,3,4,5-Tetrafluorbenzoyl)-3-(4-fluoranilino)-acrylsäureethylester (6) (R = 4-Fluorphenyl, $X^1$-$X^4$ = F) in 60 ml wasserfreiem Dimethylformamid wird mit 2,7 g Natriumfluorid versetzt. Dann wird 2 Stunden unter Rückfluß gerührt und das Reaktionsgemisch heiß auf Eis gegossen. Der Niederschlag wird abgesaugt, mit Wasser gewaschen und im Vak. über Calciumchlorid bei 100°C getrocknet. Nach Umkristallisation aus Ethanol werden 14,8 g (7) (R = 4-Fluorphenyl, $X^1$-$X^3$ = F) vom Schmelzpunkt 228 - 229°C erhalten.

Ein Gemisch von 13,6 g (7)(R = 4-Fluorphenyl, $X^1$-$X^3$ = F), 100 ml Eisessig, 75 ml Wasser und 10 ml konz. Schwefelsäure wird 2 Stunden auf Rückfluß erhitzt. Dann gießt man heiß auf Eis, saugt den Niederschlag ab, wäscht gut mit Wasser nach und trocknet im Vak. bei 100°C. Man erhält 11,7 g 6,7,8-Trifluor-1,4-dihydro-4-oxo-1-(4-fluorphenyl)-chinolin-3-carbonsäure (II) (R = 4-Fluorphenyl, $X^1$-$X^3$ = F) vom Schmelzpunkt 261 - 263°C. Nach Umkristallisation aus Acetonitril schmelzen die Kristalle bei 263 - 264°C.

Beispiel C

6,7,8-Trifluor-1,4-dihydro-4-oxo-1-phenyl-chinolin-3-carbonsäure

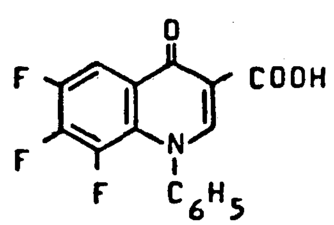

a) Analog Beispiel B (a) wird unter Verwendung von Anilin statt 4-Fluoranilin der Enaminoester (6) (R = $C_6H_5$, $X^1$-$X^4$ = F) in 85 %iger Ausbeute erhalten. Schmelzpunkt: 88 - 90°C.

b) Der Ringschluß von (6) (R = $C_6H_5$, $X^1$-$X^4$ = F) führt analog Beispiel B (b) zum 6,7,8-Trifluor-1,4-dihydro-4-oxo-1-phenyl-chinolin-3-carbonsäureethylester (7) (R = $C_6H_5$, $X^1$-$X^3$ = F) vom Schmelzpunkt 190 - 192°C.
Ausbeute: 90 %.
Die saure Verseifung von (7) (R = $C_6H_5$, $X^1$-$X^3$ = F) liefert die 6,7,8-Trifluor-1,4-dihydro-4-oxo-1-phenyl-chinolin-3-carbonsäure (II) (R = $C_6H_5$, $X^1$-$X^3$ = F) vom Schmelzpunkt 272 - 274°C.
Ausbeute: 90 %.

Beispiel D

6,7,8-Trifluor-1,4-dihydro-4-oxo-1-(4-chlor-phenyl)-chinolin-3-carbonsäure

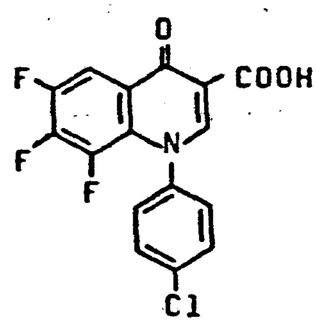

a) Analog Beispiel B (a) wird unter Verwendung von 4-Chloranilin anstelle von 4-Fluoranilin der entsprechende Enaminoester (6) (R = 4-Cl-$C_6H_4$-, $X^1$-$X^4$ = F) in 75 %iger Ausbeute erhalten. Schmelzpunkt: 117 - 118°C.

b) Analog Beispiel B (b) wird aus (6) (R = 4-Cl-$C_6H_4$-, $X^1$-$X^4$ = F) der 6,7,8-Trifluor-1,4-dihydro-4-oxo-1-(4-chlorphenyl)-chinolin-3-carbonsäureethylester (7) (R = 4-Cl-$C_6H_4$-, $X^1$-$X^3$ = F) in 85 %iger Ausbeute erhalten. Schmelzpunkt: 214 - 216°C.
Die saure Verseifung liefert die entsprechende Chinoloncarbonsäure (II) (R = 4-Cl-$C_6H_4$-, $X^1$-$X^3$ = F) in 90 %iger Ausbeute. Schmelzpunkt: 290 - 292°C.

Beispiel E

6,7,8-Trifluor-1,4-dihydro-4-oxo-1-(4-methyl-phenyl)-chinolin-3-carbonsäure

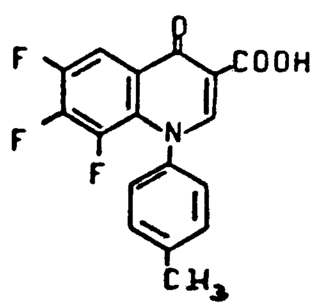

a) Analog Beispiel B (a) wird unter Verwendung von 4-Methylanilin anstelle von 4-Fluoranilin der entsprechende Enaminoester (6) (R = 4-$CH_3$-$C_6H_4$-, $X^1$-$X^4$ = F) in 82 %iger Ausbeute hergestellt. Schmelzpunkt 110 - 111°C.

b) Analog Beispiel B (b) wird aus (6) (R = 4-$CH_3$-$C_6H_4$-, $X^1$-$X^4$ = F) der entsprechende Chinoloncarbonsäure-ethylester (7) (R = 4-$CH_3$-$C_6H_4$-, $X^1$-$X^3$ = F) in 90 %iger Ausbeute erhalten.
Schmelzpunkt: 223 - 224°C.
Die saure Verseifung liefert die entsprechende Chinoloncarbonsäure (II) (R = 4-$CH_3$-$C_6H_4$-, $X^1$-$X^3$ = F) in 78 %iger Ausbeute.
Schmelzpunkt: 282 - 285°C.

Beispiel F

6,7,8-Trifluor-1,4-dihydro-4-oxo-1-(3-chlor-4-fluor-phenyl)-chinolin-3-carbonsäure

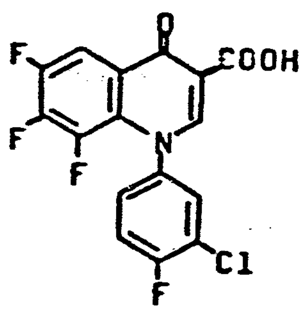

a) Analog Beispiel B (a) wird unter Verwendung von 3-Chlor-4-fluor-anilin an Stelle von 4-Fluor-anilin der entsprechende Enaminoester (6) (R = F-$C_6H_4$-, $X^1$-$X^4$ = F) in 74 %iger Ausbeute hergestellt.
Schmelzpunkt: 102 - 104°C.

b) Analog Beispiel B (b) wird aus (6) (R = F-$C_6H_4$-, $X^1$-$X^4$ = F) der entsprechende Chinoloncarbon-

säureethylester (7) (R = F-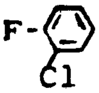-, $X^1$-$X^3$ = F) in 72 %iger Ausbeute erhalten.

Schmelzpunkt: 243 - 245°C.

Die saure Verseifung liefert die entsprechende Chinoloncarbonsäure (II) (R = F-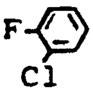, $X^1$-$X^3$ = F) in 87 %iger Ausbeute.

Schmelzpunkt: 235 - 236°C.

Beispiel G

6,7,8-Trifluor-1,4-dihydro-4-oxo-1-(3,4-difluorphenyl)-chinolin-3-carbonsäure

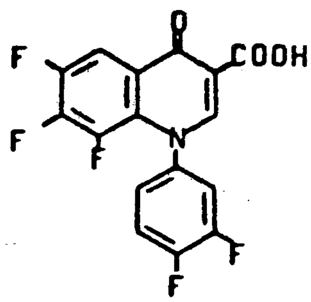

a) Analog Beispiel B (a) wird unter Verwendung von 3,4-Difluor-anilin an Stelle von 4-Fluoranilin der entsprechende Enaminoester (6) (R = F-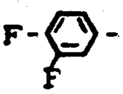-, $X^1$-$X^4$ = F) in 70 %iger Ausbeute hergestellt.

Schmelzpunkt: 87 - 90°C.

b) Analog Beispiel B (b) wird aus (6) (R = F-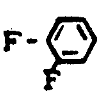-, $X^1$-$X^4$ = F) der entsprechende Chinoloncarbonsäureethylester (7) (R = F-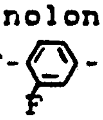-, $X^1$-$X^3$ = F) in 74 %iger Ausbeute erhalten.

Schmelzpunkt: 236 - 238°C.

Die saure Verseifung liefert die entsprechende Chinoloncarbonsäure (II) (R = F-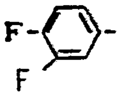-, $X^1$-$X^3$ = F) in 88 %iger Ausbeute.
Schmelzpunkt: 267 - 268°C.

Beispiel H

6,7,8-Trifluor-1,4-dihydro-4-oxo-1-(3-pyridyl)-chinolin-3-carbonsäure

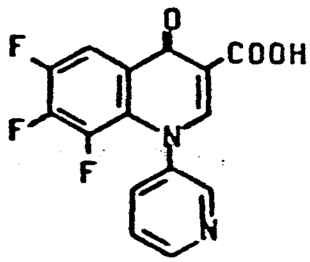

a) Analog Beispiel B (a) wird unter Verwendung von 3-Aminopyridin an Stelle von 4-Fluoranilin der entsprechende Enaminoester (6) (R = 3-Pyridyl, $X^1$-$X^4$ = F) in 65 %iger Ausbeute hergestellt. Schmelzpunkt : 122-124°C.

b) Analog Beispiel B (b) wird aus (6) (R = 3-Pyridyl, $X^1$-$X^4$ = F) der entsprechende Chinoloncarbonsäure-ethylester (R = 3-Pyridyl, $X^1$-$X^3$ = F) in 68 %iger Ausbeute erhalten. Schmelzpunkt : 198-200°C. Die saure Verseifung liefert nach folgender Neutralisation die Chinoloncarbonsäure (II) (R = 3-Pyridyl, $X^1$-$X^3$ = F) in 50 %iger Ausbeute.

Beispiel 1

6,7,8-Trifluor-1,4-dihydro-4-oxo-1-(3-fluorphenyl)-chinolin-3-carbonsäure

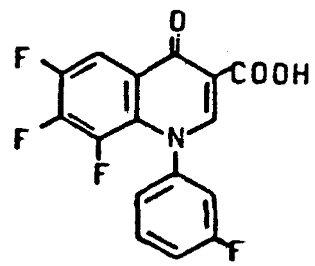

a) Analog Beispiel B (a) wird unter Verwendung von 3-Fluoranilin an Stelle von 4-Fluoranilin der entsprechende Enaminoester (6) (R = 3-Fluorphenyl, $X^1$-$X^4$ = F) in 88 %iger Ausbeute hergestellt.
Schmelzpunkt: 89 - 91°C.

b) Analog Beispiel B (b) wird aus (6) (R = 3-Fluorphenyl, $X^1$-$X^4$ = F) der entsprechende Chinolon-carbonsäureethylester (7) (R = 3-Fluorphenyl, $X^1$-$X^3$ = F) in 80 %iger Ausbeute erhalten.
Schmelzpunkt: 214 - 216°C.
Die saure Verseifung liefert die entsprechende Chinolon-carbonsäure (II) (R = 3-Fluorphenyl, $X^1$-$X^3$ = F) in 85 %iger Ausbeute.
Schmelzpunkt: 254 - 256°C.
Weiterhin können analog folgende 1,4-Dihydro-4-oxo-1-aryl-chinolin-3-carbonsäuren (II) ($X^1$-$X^3$ = F bzw. $X^1$ = Cl, $X^2$=$X^3$=F) hergestellt werden: R = 2-Fluorphenyl-, 3,5-Difluorphenyl-, 2,6-Difluorphenyl-, 2,5-Difluorphenyl-, 2,4-Difluorphenyl-, 2,4,6-Trifluorphenyl-, 2,3,4,5,6-Pentafluorphenyl-, 2-Fluor-4-methylphenyl-, 4-Fluor-3-methyl-phenyl-, 4-Fluor-2-methylphenyl-, 3-Chlor-6-fluor-phenyl-, 4-Chlor-2-fluorphenyl-, 3-Fluor-2-methyl-phenyl-, 3-Fluor-6-methylphenyl-, 3-Chlor-4,6-difluor-phenyl-, 3,5-Dichlor-4-fluor-phenyl-, 4-Fluor-3-nitro-phenyl-, 4-Methoxyphenyl-, 4-Methylmercapto-phenyl-.

Beispiel 1

6,8-Difluor-1,4-dihydro-4-oxo-1-(4-fluorphenyl)-7-(4-methyl-1-piperazinyl)-chinolin-3-carbonsäure

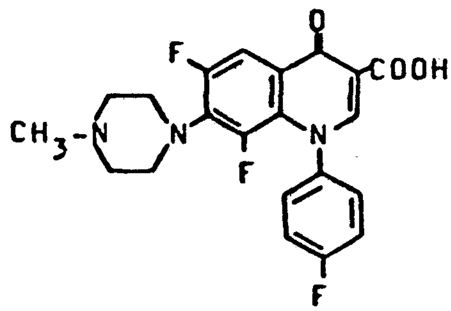

3 g 6,7,8-Trifluor-1,4-dihydro-4-oxo-1-(4-fluor-phenyl)-chinolin-3-carbonsäure werden in 30 ml Pyridin mit 2,7 g N-Methylpiperazin 6 Std. refluxiert. Das Lösungsmittel wird im Vak. abdestilliert, der Rückstand in 30 ml Wasser aufgenommen, der Niederschlag kalt abgesaugt, mit Wasser gewaschen, im Vak. über Calciumchlorid bei 100°C getrocknet und aus Glykolmonomethylether umkristallisiert.
Ausbeute: 2,5 g. Schmelzpunkt: 274 - 277°C (unter Zersetzung).
Mit konz. Salzsäure erhält man analog Beispiel 3 das Hydrochlorid.
Schmelzpunkt: >300°C (unter Zersetzung).

Beispiel 2

6,8-Difluor-1,4-dihydro-4-oxo-1-(4-fluorphenyl)-7-(3-methyl-1-piperazinyl)-chinolin-3-carbonsäure

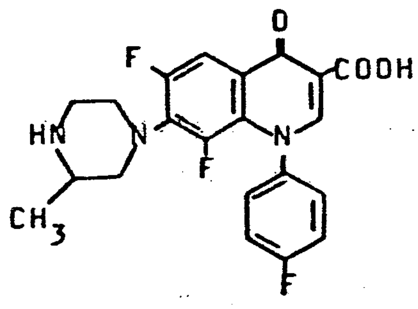

Man verfährt analog Beispiel 1, wobei mit 2-Methylpiperazin 6 Std. refluxiert und das Reaktionsprodukt aus Glykolmonomethylether umkristallisiert wird.
Ausbeute: 2 g, Schmelzpunkt: 260 - 262°C (unter Zersetzung).

Beispiel 3

6,8-Difluor-1,4-dihydro-4-oxo-1-(4-fluorphenyl)-7-(1-piperazinyl)-chinolin-3-carbonsäure

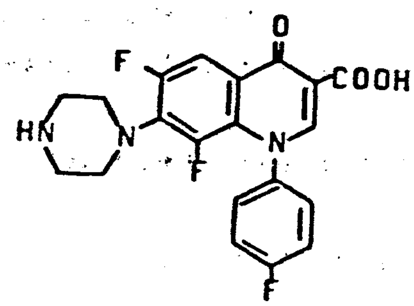

3 g 6,7,8-Trifluor-1,4-dihydro-4-oxo-1-(4-fluor-phenyl)-chinolin-3-carbonsäure werden mit 3,1 g Piperazin analog Beispiel 1 umgesetzt. Das aus Dimethylsulfoxid/Ethanol umkristallisierte Reaktionsprodukt (1,8 g vom Schmelzpunkt 272 - 276°C (unter Zersetzung)) wird mit 7,5 ml Wasser und 7,5 ml konzentrierter Salzsäure auf 90 - 100°C erhitzt und die erkaltete Suspension mit 30 ml Ethanol versetzt. Das so gebildete Hydrochlorid wird abgesaugt, mit Ethanol gewaschen und getrocknet.
Ausbeute: 1,4 g, Schmelzpunkt: 333 - 335°C (unter Zersetzung).

Beispiel 4

6,8-Difluor-1,4-dihydro-4-oxo-1-(4-fluorphenyl)-7-(4-ethyl-1-piperazinyl)-chinolin-3-carbonsäure

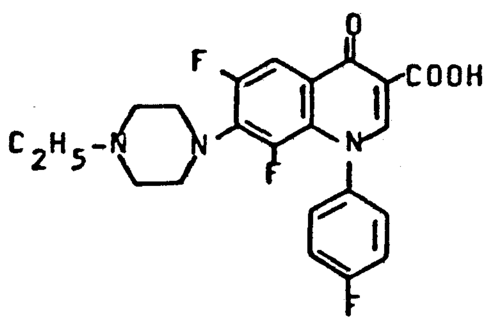

Analog Beispiel 1 wird mit N-Ethylpiperazin umgesetzt und das Reaktionsprodukt anschließend in das Hydrochlorid überführt.
Ausbeute: 2 g, Schmelzpunkt: >300°C (unter Zersetzung).

Beispiel 5

6,8-Difluor-1,4-dihydro-4-oxo-1-(4-fluorphenyl)-7-(1-pyrrolidinyl)-chinolin-3-carbonsäure

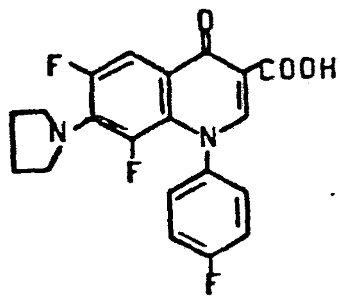

3,37 g 6,7,8-Trifluor-1,4-dihydro-4-oxo-1-(4-fluorphenyl)-chinolin-3-carbonsäure werden mit 2,3 g Pyrrolidin in 30 ml absolutem Pyridin 6 Std. refluxiert. Das Lösungsmittel wird im Vak. abdestilliert, der Rückstand in 50 ml Eiswasser suspendiert, der pH mit Salzsäure auf 1 - 2 gestellt, der Niederschlag abgesaugt, mit Wasser gewschen und getrocknet. Es werden 2,5 g (I) (R = 4-Fluorphenyl, $X^1=X^2=F$, A = 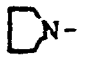) vom Schmelzpunkt 282 - 284°C erhalten. Umkristallisation aus Glykolmonomethylether/Ethanol.

Beispiel 6

6,8-Difluor-1,4-dihydro-4-oxo-1-(4-fluorphenyl)-7-(3-amino-1-pyrrolidinyl)-chinolin-3-carbonsäure

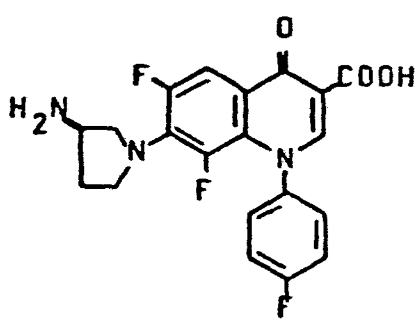

a) Eine Lösung von 3,2 g 6,7,8-Trifluor-1,4-dihydro-4-oxo-1-(4-fluorphenyl)-chinolin-3-carbonsäure in 30 ml Dimethylsulfoxid wird mit 6 g 3-Acetamido-pyrrolidin versetzt. Man erhitzt 6 Std. auf 120 - 130°C, zieht das Lösungsmittel im Vak. ab, nimmt mit Eiswasser auf, stellt mit konz. Salzsäure auf pH = 1 - 2, saugt ab und wäscht mit Wasser gut nach. Es werden 3,4 g (I) (R = 4-Fluorphenyl, $X^1$=$X^2$=F, A=$CH_3CO$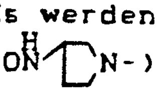) erhalten.

b) Das oben beschriebene Produkt wird mit Salzsäure bei 80 - 100°C hydrolysiert. Es werden 2,4 g 6,8-Difluor-1,4-dihydro-4-oxo-1-(4-fluorphenyl)-7-(3-amino-1-pyrrolidinyl)-chinolin-3-carbonsäure-Hydrochlorid vom Schmelzpunkt >300°C (unter Zersetzung) erhalten.

Beispiel 7

6,8-Difluor-1,4-dihydro-4-oxo-1-phenyl-7-(4-methyl-1-piperazinyl)-chinolin-3-carbonsäure

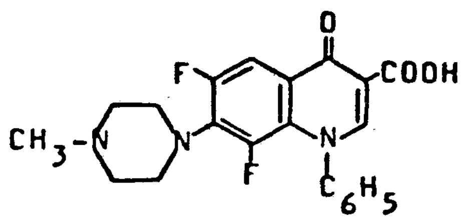

Die Herstellung erfolgt analog Beispiel 1. Als Ausgangsprodukte werden die 6,7,8-Trifluor-1,4-dihydro-4-oxo-1-phenyl-chinolin-3-carbonsäure und N-Methylpiperazin verwendet. Man erhält das 6,8-Difluor-1,4-dihydro-4-oxo-1-phenyl-7-(4-methyl-1-piperazinyl)-chinolin-3-carbonsäure-Hydrochlorid vom Schmelzpunkt >300°C (unter Zersetzung) in guter Ausbeute. Schmelzpunkt des Betains : 225-230°C.

Beispiel 8

6,8-Difluor-1,4-dihydro-4-oxo-1-phenyl-7-(1-piperazinyl)-chinolin-3-carbonsäure

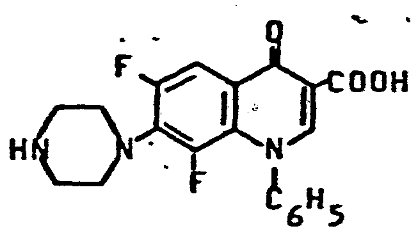

Die Herstellung erfolgt analog Beispiel 3. Als Ausgangsprodukte werden die 6,7,8-Trifluor-1,4-dihydro-4-oxo-1-phenyl-chinolin-3-carbonsäure und Piperazin verwendet. Man erhält das entsprechende Chinolin-3-carbonsäure-Hydrochlorid (I) (R = $C_6H_5$, $X^1$=$X^2$=F, A=Piperazinyl) vom Schmelzpunkt >300°C in 75 %iger Ausbeute. Schmelzpunkt des Betains: 288-300°C (unter Zersetzung).

Beispiel 9

6,8-Difluor-1,4-dihydro-4-oxo-1-phenyl-7-(3-methyl-1-piperazinyl)-chinolin-3-carbonsäure

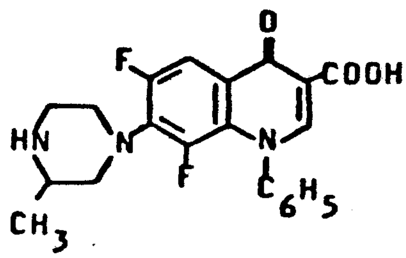

Die Synthese erfolgt analog Beispiel 1. Als Ausgangsprodukte werden die 6,7,8-Trifluor-1,4-dihydro-4-oxo-1-phenyl-chinolin-3-carbonsäure und 2-Methylpiperazin verwendet. Man erhält das entsprechende Chinolin-3-carbonsäure-Hydrochlorid (I) (R = $C_6H_5$, $X^1$=$X^2$=F, A = 2-Methyl-1-piperazinyl) vom Schmelzpunkt >300°C in 70 %iger Ausbeute.

Beispiel 10

6,8-Difluor-1,4-dihydro-4-oxo-1-phenyl-7-(4-ethyl-1-piperazinyl)-chinolin-3-carbonsäure

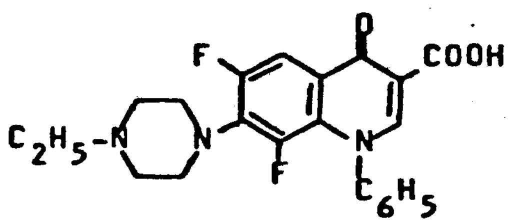

Die Synthese erfolgt analog Beispiel 1. Als Ausgangsprodukte werden die 6,7,8-Trifluor-1,4-dihydro-4-oxo-1-phenyl-chinolin-3-carbonsäure und N-Ethylpiperazin verwendet. Das entsprechende Chinolin-3-carbonsäure-Hydrochlorid (I) (R = $C_6H_5$, $X^1$=$X^2$=F, A = 4-Ethyl-1-piperazinyl) vom Schmelzpunkt >300°C wird in 60 %iger Ausbeute erhalten. Schmelzpunkt des Betains : 227-229°C.

Beispiel 11

6,8-Difluor-1,4-dihydro-4-oxo-1-(4-chlorphenyl)-7-(4-methyl-1-piperazinyl)-chinolin-3-carbonsäure

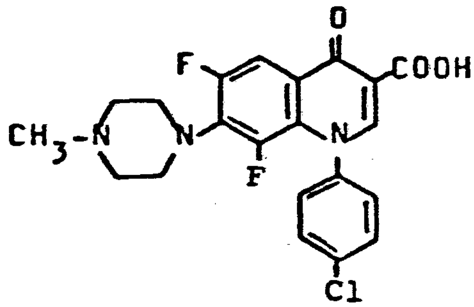

Die Synthese erfolgt analog Beispiel 1. Als Ausgangsstoffe werden die 6,7,8-Trifluor-1,4-dihydro-4-oxo-1-(4-chlorphenyl)-chinolin-3-carbonsäure und N-Methylpiperazin verwendet. Das entsprechende Chinolin-3-carbonsäure-Hydrochlorid (I) (R = 4-Cl-$C_6H_4$-, $X^1$=$X^2$=F, A = 4-Ethyl-1-piperazinyl) vom Schmelzpunkt >300°C wird in 78 %iger Ausbeute erhalten. Schmelpunkt des Betains : 274-278°C.

Beispiel 12

6-Fluor-1,4-dihydro-4-oxo-8-nitro-1-phenyl-7-(4-methyl-1-piperazinyl)-chinolin-3-carbonsäure

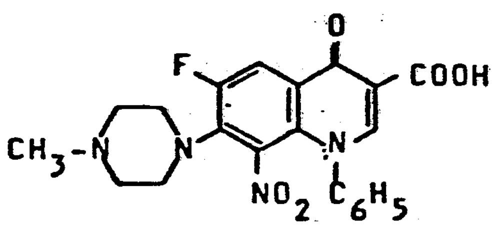

Die Synthese erfolgt analog Beispiel 1 (2 Std. Rückfluß). Als Ausgangsprodukte werden die 7-Chlor-6-fluor-1,4-dihydro-4-oxo-8-nitro-1-phenyl-chinolin-3-carbonsäure und N-Methyl-piperazine verwendet. Das als Reaktionsprodukt erhaltene Chinolin-3-carbonsäure-Hydrochlorid (I) (R = $C_6H_5$, $X^1$ = $NO_2$, $X^2$ = F, A = 4-Methyl-1-piperazinyl) besitzt einen Schmelzpunkt >300°C (unter Zersetzung). Schmelzpunkt des Betains : 240-245°C.

Beispiel 13

6,8-Difluor-1,4-dihydro-4-oxo-1-(4-methylphenyl)-7-(1-piperazinyl)-chinolin-3-carbonsäure

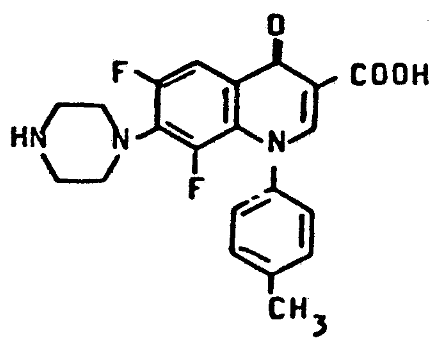

Die Synthese erfolgt analog Beispiel 3. Als Ausgangsstoffe werden die 6,7,8-Trifluor-1,4-dihydro-4-oxo-1-(4-methylphenyl)-chinolin-3-carbonsäure und Piperazin verwendet. Das als Reaktionsprodukt in guter Ausbeute erhaltene Chinolin-3-carbonsäure-Hydrochlorid (I) (R = 4-Methylphenyl, $X^1$=$X^2$=F, A = Piperazinyl) besitzt einen Schmelzpunkt >300°C.

Beispiel 14

6,8-Difluor-1,4-dihydro-4-oxo-1-(4-methylphenyl)-7-(4-ethyl-1-piperazinyl)-chinolin-3-carbonsäure

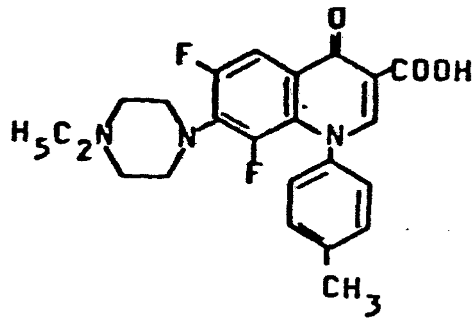

Die Synthese erfolgt analog Beispiel 1. Als Ausgangsprodukte werden die 6,7,8-Trifluor-1,4-dihydro-4-oxo-1-(4-methylphenyl)-chinolin-3-carbonsäure und N-Ethylpiperazin verwendet. Das als Reaktionsprodukt in 68 %iger Ausbeute erhaltene Chinolin-3-carbonsäure-Hydrochlorid (I) (R = 4-Methylphenyl, $X^1=X^2=F$, A = 4-Ethylpiperazinyl) besitzt einen Schmelzpunkt >300°C. Schmelzpunkt des Betains: 198-200°C.

Beispiel 15

6,8-Difluor-1,4-dihydro-4-oxo-1-(3,4-difluor-phenyl)-7-(1-pyrrolidinyl)-chinolin-3-carbonsäure

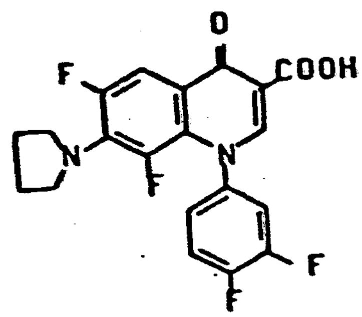

Die Synthese erfolgt analog Beispiel 5. Als Ausgangsstoffe werden die 6,7,8-Trifluor-1,4-dihydro-4-oxo-1-(3,4-difluorphenyl)-chinolin-3-carbonsäure und Pyrrolidin verwendet. Die als Reaktionsprodukt erhaltene Chinolin-3-carbonsäure (I) (R = 3,4-Difluorphenyl, $X^1$=$X^2$=F, A = Pyrrolidinyl) besitzt einen Schmelzpunkt von 295 °C.

Beispiel 16

6,8-Difluor-1,4-dihydro-4-oxo-1-(3,4-difluorphenyl)-7-(1-piperazinyl)-chinolin-3-carbonsäure

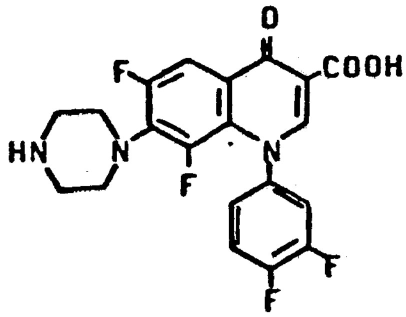

Die Synthese erfolgt analog Beispiel 3. Als Ausgangsstoffe werden die 6,7,8-Trifluor-1,4-dihydro-4-oxo-1-(3,4-difluorphenyl)-chinolin-3-carbonsäure und Piperazin verwendet. Das als Reaktionsprodukt erhaltene Chinolin-3-carbonsäure-Hydrochlorid (I) (R = 3,4-Dichlorphenyl, $X^1$=$X^2$=F, A = Piperazinyl) besitzt einen Schmelzpunkt >300°C.

Beispiel 17

6,8-Difluor-1,4-dihydro-4-oxo-1-(3-fluorphenyl)-7-(4-methyl-1-piperazinyl)-chinolin-3-carbonsäure

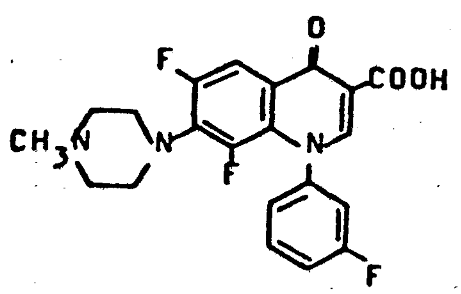

Die Synthese erfolgt analog Beispiel 1. Als Ausgangsstoffe werden die 6,7,8-Trifluor-1,4-dihydro-4-oxo-1-(3-fluorphenyl)-chinolin-3-carbonsäure und N-Methylpiperazin verwendet. Das als Reaktionsprodukt erhaltene Chinolin-3-carbonsäure-Hydrochlorid (I) (R = 3-Fluorphenyl, $X^1$=$X^2$=F, A = 4-Methylpiperazinyl) besitzt einen Schmelzpunkt >300°C.

Analog können folgende in 7-Stellung substituierte Chinolin-3-carbonsäuren und die entsprechenden Hydrochloride (I) hergestellt werden (Schmelzpunkte der Hydrochloride >300°C):

| Beispiel | R | $X^1$ | $X^2$ | A |
|---|---|---|---|---|
| 18 | 4-Chlorphenyl | F | F | 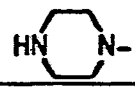 |
| 19 | 4-Chlorphenyl | F | F | 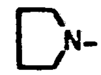 |
| 20 | 4-Methylphenyl | F | F | 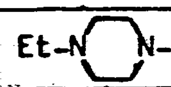 |
| 21 | 3-Chlor-4-fluorphenyl | F | F | 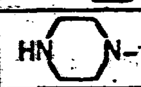 |
| 22 | 3-Chlor-4-fluorphenyl | Cl | F | 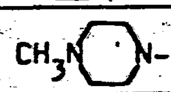 Betain: (Fp.268°C) |
| 23 | 3,4-Difluorphenyl | F | F | 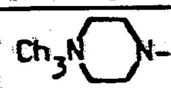 |
| 24 | 3,4-Difluorphenyl | F | F | 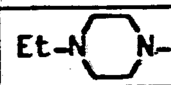 |
| 25 | 3,4-Difluorphenyl | Cl | F | 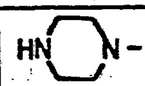 |
| 26 | 3,4-Difluorphenyl | F | F | 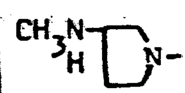 |
| 27 | 3-Fluorphenyl | F | F | 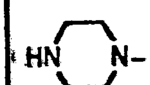 |
| 28 | 3-Fluorphenyl | F | F | 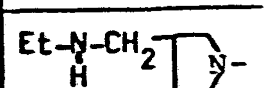 |
| 29 | 3-Fluorphenyl | Cl | F | 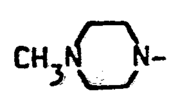 |

| Beispiel | R | $X^1$ | $X^2$ | A |
|---|---|---|---|---|
| 30 | 3-Pyridyl | F | F | 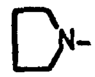 |
| 31 | 3-Pyridyl | F | F | 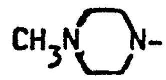 |
| 32 | 2-Fluorphenyl | F | F | 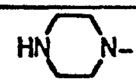 |
| 33 | 2-Fluorphenyl | F | F | 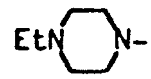 |
| 34 | 2-Fluorphenyl | F | F | 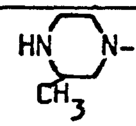 |
| 35 | 2-Fluorphenyl | Cl | F | 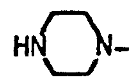 |
| 36 | 3,5-Difluorphenyl | F | F | 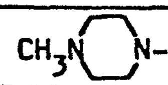 |
| 37 | 2,6-Difluorphenyl | F | F | 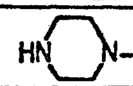 |
| 38 | 4-Fluor-3-methylphenyl | F | F | 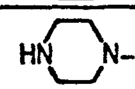 |
| 39 | 4-Fluor-3-methylphenyl | F | F | 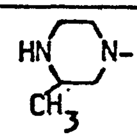 |
| 40 | 4-Fluor-3-methylphenyl | F | F | 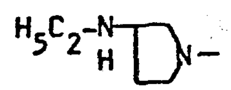 |
| 41 | 4-Fluor-3-methylphenyl | F | F | 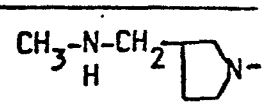 |
| 42 | 4-Methoxyphenyl | F | F | 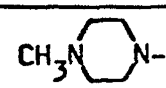 |
| 43 | 4-Methoxyphenyl | Cl | F | 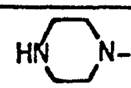 |
| 44 | 4-Methylmercaptophenyl | F | F | 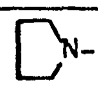 |
| 45 | 4-Methylmercaptophenyl | F | F | 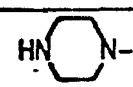 |

Beispiel 46

6,8-Difluor-1,4-dihydro-4-oxo-1-(4-fluorphenyl)-7-[4-(2-oxo-propyl)-1-piperazinyl]-chinolin-3-carbonsäure-Hydrochlorid

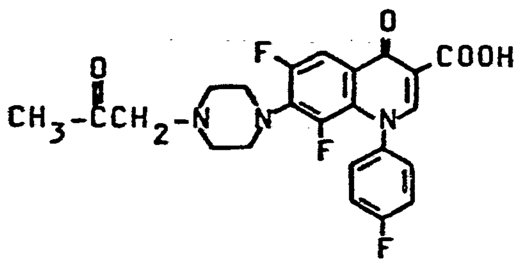

x HCl

2 g 6,8-Difluor-1,4-dihydro-4-oxo-1-(4-fluorphenyl)-7-(1-piperazinyl)-chinolin-3-carbonsäure werden in 25 ml Dimethylformamid mit 0,7 g Chloraceton und 1,05 g Triethylamin versetzt und 3 Std. auf 80°C erhitzt. Die Suspension wird im Vakuum eingeengt, der Rückstand mit Wasser verrührt, abgesaugt und getrocknet. Das Produkt wird in 15 ml verdünnter Salzsäure (1:1) erhitzt, mit 30 ml Ethanol ausgefällt, abgesaugt und getrocknet.
Ausbeute: 1,9 g, Schmelzpunkt: >300°C (unter Zersetzung).

Beispiel 47

6,8-Difluor-1,4-dihydro-4-oxo-1-(4-fluorphenyl)-7-[4-(3-oxo-butyl)-1-piperazinyl]-chinolin-3-carbonsäure-Hydrochlorid

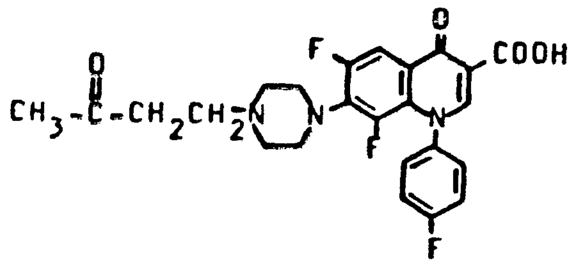

x HCl

2 g 6,8-Difluor-1,4-dihydro-4-oxo-1-(4-fluorphenyl)-7-(1-piperazinyl)-chinolin-3-carbonsäure und 1,95 g Methylvinylketon werden in 25 ml Ethanol 7 Std. unter Rückfluß erhitzt, der erhaltene Niederschlag abgesaugt, in verdünnter Salzsäure (1:1) gelöst und mit Ethanol ausgefällt. Ausbeute: 1,6 g, Schmelzpunkt >300°C (unter Zersetzung).

## Patentansprüche

1. 1-Aryl-4-chinolon-3-carbonsäuren der Formel (I),

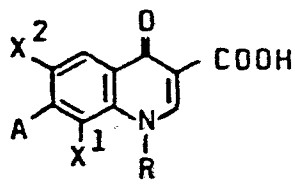

(I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen, Alkoxy, Alkylmercapto oder Alkylsulfonyl mit jeweils bis zu 3 Kohlenstoffatomen, Nitro, Cyano, Carboxyl, Methylendioxy sowie einen Aminrest

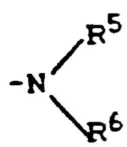

,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeuten, wobei das Heteroatom S, O oder N sein kann und

A für 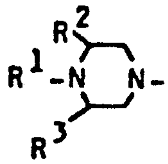 oder 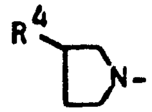 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 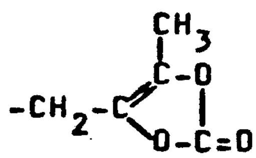

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

und deren pharmazeutisch verwendbare Hydrate, Säureadditionssalze, Alkali-, Erdalkali-, Silber- und Guanidiniumsalze, sowie in Form ihrer Ester und in anderen üblichen Prodrug-Formen.

2. 1-Aryl-4-chinolon-3-carbonsäuren der Formel (I) gemäß Anspruch 1, in der

$X^1$ für Chlor, Fluor oder Nitro,

$X^2$ für Chlor oder Fluor,

R für einen gegebenenfalls durch Halogen, Alkyl, Alkoxy, Alkylmercapto oder Alkylsulfonyl mit jeweils bis zu 2 Kohlenstoffatomen im Alkylteil, Nitro, Cyano substituierten Phenylrest oder einen Pyridin-, Thiophen-, Furan- oder Thiazolrest steht und

A für 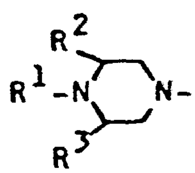 oder 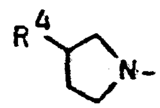 oder Halogen,

insbesondere Chlor oder Fluor steht, worin

$R^1$ für Wasserstoff, eine verzweigte oder unverzweigte Alkylgruppe mit 1 bis 3 Kohlen-

stoffatomen, die gegebenenfalls durch eine Hydroxygruppe substituiert sein kann, einen gegebenenfalls durch Chlor oder Fluor substituierten Phenacylrest, einen Oxoalkylrest mit 4 oder 4 Kohlenstoffatomen, 4-Aminobenzyl, Formyl oder Acetyl,

$R^2$ für Wasserstoff, Methyl oder gegebenenfalls durch Chlor oder fluor substituiertes Phenyl,

$R^3$ für Wasserstoff oder Methyl und

$R^4$ für Wasserstoff, Hydroxy, Amino, Methylamino, Ethylamino, Aminomethyl, Methylaminomethyl, Ethylaminoethyl oder Diethylaminomethyl steht.

3. 1-Aryl-4-chinolon-3-carbonsäuren der Formel (I) gemäß Anspruch 1, in der

$X^1$ für Chlor oder Fluor,

$X^2$ für Fluor,

R für einen gegebenenfalls durch Chlor oder Fluor, Methyl, Methoxy, Methylmercapto, Nitro, Cyano substituierten Phenylrest steht, und

A für 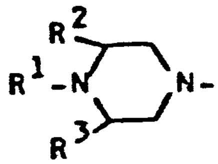 oder 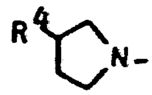 oder Halogen.

insbesondere Chlor oder Fluor steht, worin

$R^1$ für Wasserstoff, Methyl, Ethyl, 2-Hydroxyethyl, Phenacyl, 2-Oxopropyl, 3-Oxobutyl oder Formyl,

$R^2$ für Wasserstoff, Methyl oder Phenyl,

$R^3$ für Wasserstoff oder Methyl und

$R^4$ für Wasserstoff, Amino, Methylamino, Aminomethyl, Ethylaminomethyl oder Diethylaminomethyl steht.

4. 1-Aryl-4-chinolon-3-carbonsäuren der Formel (I) gemäß den Ansprüchen 1 bis 3 in Form ihrer Methyl-, Ethyl-, Pivaloyloxymethyl-, Pivaloyl-oxyethyl- oder (5-Methyl-2-oxo-1,3-dioxol-4-yl-methyl)-ester.

5. 1-Aryl-4-chinolon-3-carbonsäuren der Formel (I),

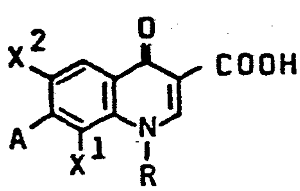 (I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen, Alkoxy, Alkylmercapto oder Alkylsulfonyl mit jeweils bis zu 3 Kohlenstoffatomen, Nitro, Cyano, Carboxyl, Methylendioxy sowie einen Aminrest

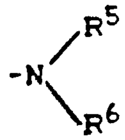 ,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 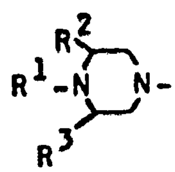 oder 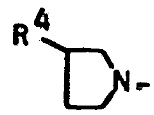 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert

sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 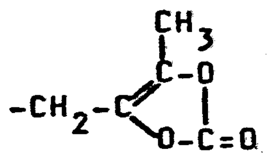

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

und deren pharmazeutisch verwendbare Hydrate, Säureadditionssalze, Alkali-, Erdalkali-, Silber- und Guanidiniumsalze, sowie in Form ihrer Ester und in anderen üblichen Prodrug-Formen zur Anwendung bei der therapeutischen Behandlung des menschlichen oder tierischen Körpers.

6. 1-Aryl-4-chinolon-3-carbonsäuren aus der Gruppe bestehend aus
6,8-Difluor-1,4-dihydro-4-oxo-1-(4-fluorphenyl)-7-(4-methyl-1-piperazinyl)-chinolin-3-carbonsäure,
6,8-Difluor-1,4-dihydro-4-oxo-1-(4-fluorphenyl)-7-(3-methyl-1-piperazinyl)-chinolin-3-carbonsäure,
6,8-Difluor-1,4-dihydro-4-oxo-1-(4-fluorphenyl)-7-(1-piperazinyl)-chinolin-3-carbonsäure,
6,8-Difluor-1,4-dihydro-4-oxo-1-(4-fluorphenyl)-7-(4-ethyl-1-piperazinyl)-chinolin-3-carbonsäure,
6,8-Difluor-1,4-dihydro-4-oxo-1-(4-fluorphenyl)-7-(1-pyrrolidinyl)-chinolin-3-carbonsäure,
6,8-Difluor-1,4-dihydro-4-oxo-1-(4-fluorphenyl)-7-(3-amino-1-pyrrolidinyl)-chinolin-3-carbonsäure,
6,8-Difluor-1,4-dihydro-4-oxo-1-phenyl-7-(4-methyl-1-piperazinyl)-chinolin-3-carbonsäure,
6,8-Difluor-1,4-dihydro-4-oxo-1-phenyl-7-(1-piperazinyl)-chinolin-3-carbonsäure,
6,8-Difluor-1,4-dihydro-4-oxo-1-phenyl-7-(3-methyl-1-piperazinyl)-chinolin-3-carbonsäure,
6,8-Difluor-1,4-dihydro-4-oxo-1-phenyl-7-(4-ethyl-1-piperazinyl)-chinolin-3-carbonsäure,
6,8-Difluor-1,4-dihydro-4-oxo-1-(4-chlorphenyl)-7-(4-methyl-1-piperazinyl)-chinolin-3-carbonsäure,
6-Fluor-1,4-dihydro-4-oxo-8-nitro-1-phenyl-7-(4-methyl-1-piperazinyl)-chinolin-3-carbonsäure,
6,8-Difluor-1,4-dihydro-4-oxo-1-(4-methylphenyl)-7-(1-piperazinyl)-chinolin-3-carbonsäure,
6,8-Difluor-1,4-dihydro-4-oxo-1-(4-methylphenyl)-7-(4-ethyl-1-piperazinyl)-chinolin-3-carbonsäure,

6,8-Difluor-1,4-dihydro-4-oxo-1-(3,4-difluor-phenyl)-7-(1-pyrrolidinyl)-chinolin-3-carbonsäure,
6,8-Difluor-1,4-dihydro-4-oxo-1-(3,4-difluorphenyl)-7-(1-piperazinyl)-chinolin-3-carbonsäure,
6,8-Difluor-1,4-dihydro-4-oxo-1-(3-fluorphenyl)-7-(4-methyl-1-piperazinyl)-chinolin-3-carbonsäure,
6,8-Difluor-1,4-dihydro-4-oxo-1-(4-fluorphenyl)-7-[4-(2-oxo-propyl)-1-piperazinyl]-chinolin-3-carbonsäure-Hydrochlorid und
6,8-Difluor-1,4-dihydro-4-oxo-1-(4-fluorphenyl)-7-[4-(3-oxo-butyl)-1-piperazinyl]-chinolin-3-carbonsäure-Hydrochlorid.

7. 1-Aryl-4-chinolon-3-carbonsäuren aus der Gruppe bestehend aus
6,8-Difluor-1,4-dihydro-4-oxo-1-(4-fluorphenyl)-7-(3,5-dimethyl-1-piperazinyl)-chinolin-3-carbonsäure,
6,8-Difluor-1,4-dihydro-4-oxo-1-(3-fluorphenyl)-7-(3,5-dimethyl-1-piperazinyl)-chinolin-3-carbonsäure,
6,8-Difluor-1,4-dihydro-4-oxo-1-(2-fluorphenyl)-7-(3,5-dimethyl-1-piperazinyl)-chinolin-3-carbonsäure,
6,8-Difluor-1,4-dihydro-4-oxo-1-(2,4-difluorphenyl)-7-(1-piperazinyl)-chinolin-3-carbonsäure,
6,8-Difluor-1,4-dihydro-4-oxo-1-(2,4-difluorphenyl)-7-(4-methyl-1-piperazinyl)-chinolin-3-carbonsäure,
6,8-Difluor-1,4-dihydro-4-oxo-(2,4-difluorphenyl)-7-(3-methyl-1-piperazinyl)-chinolin-3-carbonsäure,
6,8-Difluor-1,4-dihydro-4-oxo-1-(2,4-difluorphenyl)-7-pyrrolidinyl-chinolin-3-carbonsäure,

6,8-Difluor-1,4-dihydro-4-oxo-1-(2,4-difluorphenyl)-7-(3-amino-1-pyrrolidinyl)-chinolin-3-carbonsäure,
6,8-Difluor-1,4-dihydro-4-oxo-1-(2,4-difluor-phenyl)-7-(3-methylamino-1-pyrrolidinyl)-chinolin-3-carbonsäure,
6,8-Difluor-1,4-dihydro-4-oxo-1-(2,4-difluorphenyl)-7-(3-ethylamino-1-pyrrolidinyl)-chinolin-3-carbonsäure,
6,8-Difluor-1,4-dihydro-4-oxo-1-(2,4-difluorphenyl)-7-(3-dimethylamino-1-pyrrolidinyl)-chinolin-3-carbonsäure,
6,8-Difluor-1,4-dihydro-4-oxo-1-(2,4-difluorphenyl)-7-(3-ethylamino-methyl-1-pyrrolidinyl)-chinolin-3-carbonsäure,
8-Chlor-6-fluor-1,4-dihydro-4-oxo-1-phenyl-7-(1-piperazinyl)-chinolin-3-carbonsäure,
8-Chlor-6-fluor-1,4-dihydro-4-oxo-1-phenyl-7-(4-methyl-1-piperazinyl)-chinolin-3-carbonsäure,
8-Chlor-6-fluor-1,4- dihydro-4-oxo-1-phenyl-7-(3-methyl-1-piperazinyl)-chinolin-3-carbonsäure,
8-Chlor-6-fluor-1,4-dihydro-4-oxo-1-phenyl-7-pyrrolidinyl-chinolin-3-carbonsäure,
8-Chlor-6-fluor-1,4-dihydro-4-oxo-1-phenyl-7-(3-methylamino-1-pyrrolidinyl)-chinolin-3-carbonsäure,
8-Chlor-6-fluor-1,4-dihydro-4-oxo-1-phenyl-7-(3-ethylaminomethyl-1-pyrrolidinyl)-chinolin-3-carbonsäure und
6-Fluor-1,4-dihydro-8-nitro-4-oxo-1-phenyl-7-(4-ethyl-1-piperazinyl)-chinolin-3-carbonsäure.

8. Verbindungen der Formel (VII)

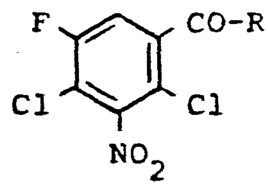

in der

R Hydroxyl, Halogen, insbesondere Chlor, oder Alkoxycarbonylmethyl mit 1 bis 4 Kohlenstoffatomen im Alkylteil bedeutet.

9. Verfahren zur Herstellung von 1-Aryl-4-chinolon-3-carbonsäuren der Formel (I),

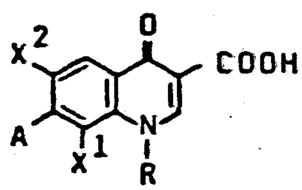

(I)

in welcher

$X^1$ für Halogen oder Nitro,

$X^2$ für Halogen, insbesondere Fluor steht.

R einen gegebenenfalls ein- oder mehrfach durch Halogenatome, Alkyl mit 1 - 4 Kohlenstoffatomen, Alkoxy, Alkylmercapto oder Alkylsulfonyl mit

jeweils bis zu 3 Kohlenstoffatomen, Nitro, Cyano, Carboxyl, Methylendioxy sowie einen Aminrest

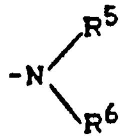 ,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 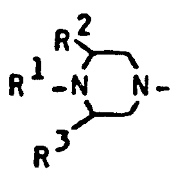 oder 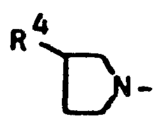 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 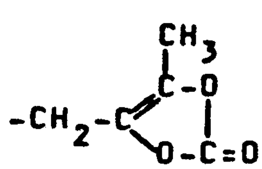 .

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

dadurch gekennzeichnet, daß man 7-Halogen-4-chinolon-3-carbonsäuren der Formel (II),

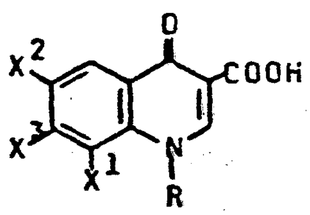

(II)

in welcher

R, $X^1$ und $X^2$ die oben angegebene Bedeutung haben. und

$X^3$ für Halogen, bevorzugt Chlor oder Fluor steht. mit Aminen der Formel (III)

A - H (III)

in welcher

A die oben angegebene Bedeutung hat,

gegebenenfalls in Gegenwart von Säurebindern umsetzt.

10. Verfahren zur Herstellung von 1-Aryl-4-chinolon-3-carbonsäuren der Formel (I)

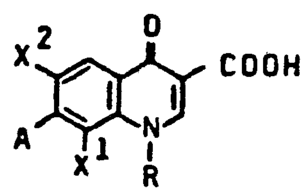

(I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen. Alkoxy, Alkylmercapto oder Alkylsulfonyl mit jeweils bis zu 3 Kohlenstoffatomen, Nitro, Cyano, Carboxyl, Methylendioxy sowie einen Aminrest

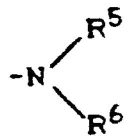 .

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 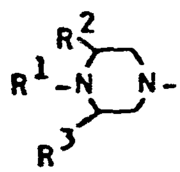 oder 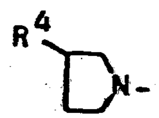 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 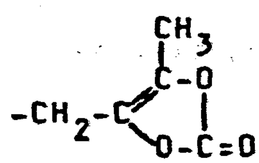

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

dadurch gekennzeichnet, daß man 1,4-Dihydro-4-oxo-7-(1-piperazinyl)-chinolin-3-carbonsäuren der Formel (IV),

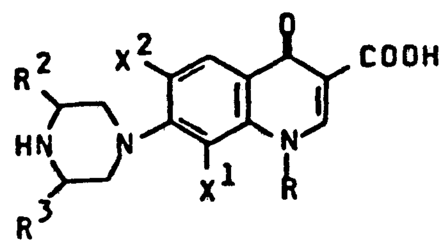

(IV)

in welcher

$X^1$, $X^2$, R, $R^2$ und $R^3$ die oben angegebene Bedeutung haben,

mit Verbindungen der Formel (V),

$$R^1 - Z \qquad (V)$$

in welcher

$R^1$ die oben angegebene Bedeutung hat, jedoch nicht Wasserstoff sein kann, und

Z Halogen, insbesondere Chlor, Brom oder Iod, Acyloxy, Ethoxy oder Hydroxy

bedeutet,

gegebenenfalls in Gegenwart von Säurebindern umsetzt.

11. Verfahren zur Herstellung von 1-Aryl-4-chinolon-3-carbonsäuren der Formel (I),

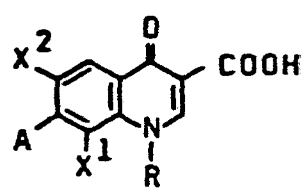

(I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen, Alkoxy, Alkylmercapto oder Alkylsulfonyl mit jeweils bis zu 3 Kohlenstoffatomen, Nitro, Cyano, Carboxyl, Methylendioxy sowie einen Aminrest

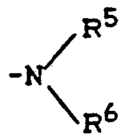 ,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 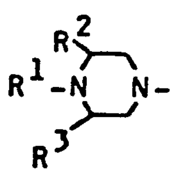 oder 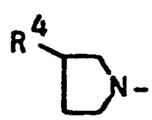 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit

2 bis 4 Kohlenstoffatomen, 4-Aminobenzyl, Formyl oder Acetyl,

oder für den Rest 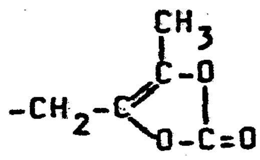

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

dadurch gekennzeichnet, daß man eine Verbindung der Formel (IV) nach Anspruch 10 mit Methylvinylketon der Formel (VI)

$$CH_3 - CO - CH = CH_2 \qquad (VI)$$

umsetzt.

12. Arzneimittel, enthaltend 1-Aryl-4-chinolin-3-carbonsäuren der Formel (I),

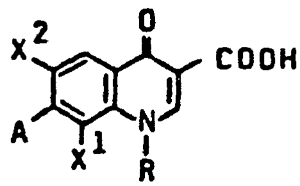

(I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen, Alkoxy, Alkylmercapto oder Alkylsulfonyl mit jeweils bis zu 3 Kohlenstoffatomen, Nitro, Cyano, Carboxyl, Methylendioxy sowie einen Aminrest

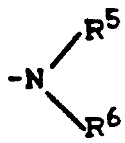

,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 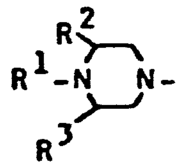 oder 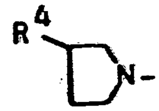 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 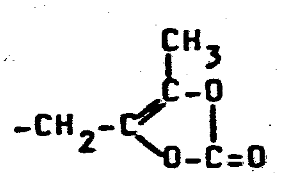

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

und deren pharmazeutisch verwendbare Hydrate, Säureadditionssalze, Alkali-, Erdalkali-, Silber- und Guanidiniumsalze, sowie in Form ihrer Ester und in anderen üblichen Prodrug-Formen.

13. Verwendung von 1-Aryl-4-chinolon-3-carbonsäuren der Formel (I),

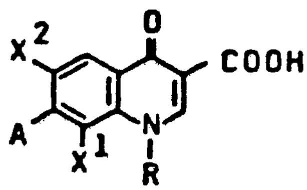

(I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen, Alkoxy, Alkylmercapto oder Alkylsulfonyl mit jeweils bis zu 3 Kohlenstoffatomen, Nitro, Cyano, Carboxyl, Methylendioxy sowie einen Aminrest

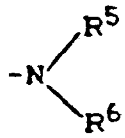,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 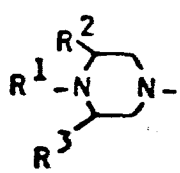 oder 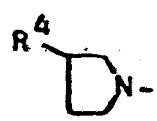 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 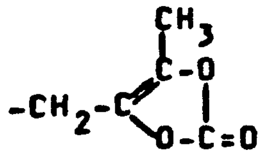

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

und deren pharmazeutisch verwendbare Hydraten, Säureadditionssalzen, Alkali-, Erdalkali-, Silber- und Guanidiniumsalzen, sowie in Form ihrer Ester und in anderen üblichen Prodrug-Formen zur Herstellung von Arzneimitteln.

14. Verwendung von 1-Aryl-4-chinolon-3-carbonsäuren der Formel (I),

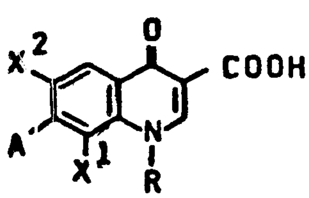

(I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen, Alkoxy, Alkylmercapto oder Alkylsulfonyl mit jeweils bis zu 3 Kohlenstoffatomen, Nitro, Cyano, Carboxyl, Methylendioxy sowie einen Aminrest

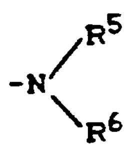 ,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 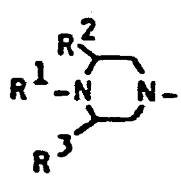 oder 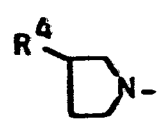 oder Halogen,

insbesondere Chlor oder Fluor steht, worin

$R^1$ für Wasserstoff, eine verzweigte oder unverzweigte Alkylgruppe mit 1 bis 4 Kohlen-

stoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 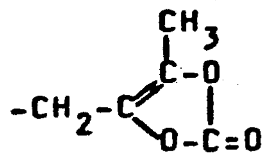

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

und deren pharmazeutisch verwendbare Hydraten, Säureadditionssalzen, Alkali-, Erdalkali-, Silber- und Guanidiniumsalzen, sowie in Form ihrer Ester und in anderen üblichen Prodrug-Formen als Wachstumsförderer in der Tierernährung.

15. Tierfutter, Tierfutterzusatzstoffe und Tierfutter-Prämixe enthaltend 1-Aryl-4-chinolon-3-carbonsäuren der Formel (I),

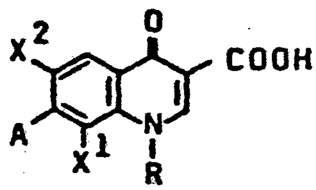

(I)

in welcher

$X^1$ für Halogen oder Nitro,

$X^2$ für Halogen, insbesondere Fluor steht,

R einen gegebenenfalls ein- oder mehrfach durch Halogenatome, Alkyl mit 1 - 4 Kohlenstoffatomen, Alkoxy, Alkylmercapto oder Alkylsulfonyl mit jeweils bis zu 3 Kohlenstoffatomen, Nitro, Cyano, Carboxyl, Methylendioxy sowie einen Aminrest

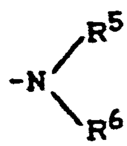 ,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 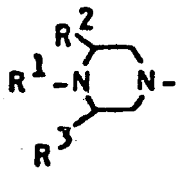 oder 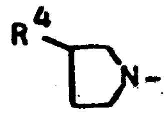 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest 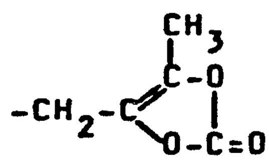

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxy-

0201829

methyl, Aminomethyl, Alkyl- oder Dialkylaminomethyl mit 1 oder 2 Kohlenstoffatomen in der Alkylgruppe steht,

und deren pharmazeutisch verwendbare Hydrate, Säureadditionssalze, Alkali-, Erdalkali-, Silber- und Guanidiniumsalze, sowie in Form ihrer Ester und in anderen üblichen Prodrug-Formen.

Europäisches Patentamt

# EUROPÄISCHER RECHERCHENBERICHT

Nummer der Anmeldung 0201829

EP 86106115.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X,P | EP - A1 - 0 154 780 (ABOTT)<br>* Ansprüche 1,10,11,13,20-22 *<br>-- | 1-3,5,12 | C 07 D 215/56<br>C 07 D 401/04<br>C 07 D 401/02 |
| A | EP - A1 - 0 131 839 (ABOTT)<br>* Ansprüche 1,9,19 *<br>-- | 1,9,12 | C 07 D 401/14<br>C 07 C 79/46<br>C 07 C 79/36 |
| A | DE - A1 - 3 106 013 (KYORIN)<br>* Ansprüche 1,17,18,20 *<br>---- | 1,9,10,12 | A 23 K 1/00<br>A 61 K 31/47 |

RECHERCHIERTE SACHGEBIETE (Int. Cl.4)

C 07 D 215/00
C 07 D 401/00
C 07 C 79/00

Der vorliegende Recherchenbericht wurde für alle Patentansprüche erstellt.

| Recherchenort | Abschlußdatum der Recherche | Prüfer |
|---|---|---|
| WIEN | 12-08-1986 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument